# EUROPEAN PATENT APPLICATION

(11) **EP 4 778 489 A1**
(43) Date of publication of application: **22.07.2026**
(21) Application number: 26151776.7
(22) Date of filing: 14.01.2026
(51) Int. Cl.: A61B 34/37, A61B 34/30, A61B 34/10

(54) **COLLABORATIVE SURGICAL/MEDICAL ROBOTIC SYSTEMS EMPLOYING MACHINE LEARNING TO CHARACTERIZE USER INTERACTION STYLE**

(30) Priority: 20.01.2025 US 202563747110 P; 19.02.2025 US 202563760142 P
(71) Applicant: MAKO Surgical Corp., Weston, FL 33331 (US)
(72) Inventor: TALASAZ, Ali, Plantation, FL 33325-2514 (US)
(74) Representative: Röthinger, Rainer

(57) **Abstract**

Surgical or medical systems and methods involve a manipulator to facilitate collaborative interaction with a human. A control system is coupled to the manipulator and is configured to acquire robotic data from the manipulator during the collaborative interaction. The control system implements a machine learning model that is configured to receive and process the robotic data to determine an interaction style of the human in collaborating with the manipulator. The control system customizes a physical feel of the manipulator based on the interaction style.

## Description

### CROSS-REFERENCE TO RELATED APPLICATIONS

The subject application claims priority to and all the benefits of United States Provisional Patent App. No. 63/747,110 filed January 20, 2025 and United States Provisional Patent App. No. 63/760,142 filed February 19, 2025, the entire contents of each of the aforementioned applications being hereby incorporated by reference.

### BACKGROUND

Collaborative robots are increasingly utilized in industries to provide an environment wherein a human user directly interacts with the robot to perform a given task. This collaborative interaction can enhance the user experience and potentially provide a more interactive working environment for the human user.

One type of collaborative robot is a surgical robot used to perform a surgical procedure. In such situations, the surgeon directly interacts with the surgical robot (or end effector attached to the surgical robot) by applying a force to move the robot to manipulate a patient tissue. During the interaction, the surgical robot may move according to a predefined setting that defines the "feel" of the robot, e.g., how sluggishly/responsively the robot reacts to the applied force. However, a predefined arm feel setting may not suit all surgeons. For example, some surgeons may be heavy-handed, while others are light-handed. If a light-handed surgeon interacts with an overly sluggish robot, the surgeon may become fatigued or frustrated during the procedure. On the other hand, if a heavy-handed surgeon interacts with a highly responsive robot, the surgeon may apply excessive force to the robot, thereby potentially resulting in arm instability, vibrations, and tool inaccuracies. Accuracy is immensely important when manipulating patient tissue. Inaccuracies of even a few millimeters can result in a sub-optimal surgical outcome for the patient.

Typical control systems for collaborative surgical robots are susceptible to these issues because they lack knowledge about the collaborative interaction style of the surgeon and are not adapted to dynamically optimize the "feel" of the robot based on the surgeon interaction style, or the context in which the surgeon is operating or collaborating with the robot.

Another type of collaborative implementation is a robotic-assisted rehabilitation platform to assist patients with improving mobility or functional strength by providing robotically guided movements or exercises. In such situations, a patient directly interacts with the rehabilitation robot (or limb holder attached to the robot) by applying a force to move the robot and perform the guided movement or exercise. Here, the robot may move according to a predefined setting that defines the "feel" of the robot, the level of robotic engagement, as well as the intensity of the therapeutic actions. Typically, these settings are defined based on a trial-and-error approach requiring verbal communication between the therapist and the patient. However, a predefined setting for a collaborative rehabilitative robot may not suit all patients. Some patients may have greater functional strength, range of movement, or pain tolerance than other patients. For example, if a functionally strong patient interacts with an overly responsive robot, the patient may not realize the full therapeutic benefits provided by the robotic guided movement thereby delaying recovery time. On the other hand, if a functional weak patient interacts with a highly resistive robot, the patient may overly exert themselves, potentially reaggravating injury.

Conventional control systems for collaborative rehabilitation robots are susceptible to these issues because they lack knowledge about the collaborative interaction style of the patient and are not adapted to dynamically optimize the "feel" of the robot based on the patient interaction style, the functional strength of the patient, or the context in which the robot is collaborating with the patient.

### SUMMARY

This Summary introduces a selection of concepts in a simplified form that are further described below in the Detailed Description below. This Summary is not intended to limit the scope of the claimed subject matter nor identify key features or essential features of the claimed subject matter.

According to a first aspect, a surgical or medical system is provided, comprising: a manipulator to facilitate collaborative interaction with a human; and a control system coupled to the manipulator and being configured to: implement a machine learning model that is configured to determine an interaction style of the human in collaborating with the manipulator; and customize a physical feel of the manipulator based on the interaction style.

According to a second aspect, a surgical or medical system is provided, comprising: a manipulator comprising a robotic arm including a plurality of links and joints and an end effector supported and moveable by the robotic arm, wherein the end effector is adapted to facilitate collaborative interaction between a human and the manipulator; and a control system coupled to the manipulator and being configured to: control the manipulator according to a behavior policy that defines a physical feel of the manipulator for the human; acquire robotic data; implement a machine learning model that is configured to receive and process the robotic data to determine an interaction style of the human in collaborating with the manipulator; update the behavior policy based on the determined interaction style to customize the physical feel of the manipulator to the determined interaction style; and control the manipulator according to the updated behavior policy.

According to a third aspect, a surgical or medical system is provided, comprising: a manipulator comprising a robotic arm including a plurality of links and joints and an end effector supported and moveable by the robotic arm, wherein the end effector is adapted to facilitate collaborative interaction between a human and the manipulator; and a control system coupled to the manipulator and being configured to: control the manipulator according to haptic forces that define a physical feel of the manipulator for the human; acquire robotic data; implement a machine learning model that is configured to receive and process the robotic data to determine an interaction style of the human in collaborating with the manipulator; refine the haptic forces based on the determined interaction style to customize the physical feel of the manipulator to the determined interaction style; and control the manipulator according to the refined haptic forces.

According to a fourth aspect, a surgical or medical system is provided, comprising: a manipulator comprising a robotic arm including a plurality of links and joints and an end effector supported and moveable by the robotic arm, wherein the end effector is adapted to facilitate collaborative interaction between a human and the manipulator; and a control system coupled to the manipulator and being configured to: acquire robotic data; implement a machine learning model that is configured to receive and process the robotic data to determine an interaction style of the human in collaborating with the manipulator; define a behavior policy based on the determined interaction style, wherein the behavior policy defines a physical feel of the manipulator; and control the manipulator according to the behavior policy.

According to a fifth aspect, a surgical or medical system is provided, comprising: a manipulator comprising a robotic arm including a plurality of links and joints and an end effector supported and moveable by the robotic arm, wherein the end effector is adapted to facilitate collaborative interaction between a human and the manipulator; and a control system coupled to the manipulator and being configured to: acquire robotic data; implement a machine learning model that is configured to receive and process the robotic data to determine an interaction style of the human in collaborating with the manipulator; define haptic forces based on the determined interaction style, wherein the haptic forces define a physical feel of the manipulator for the human; and control the manipulator according to the haptic forces.

According to a sixth aspect, a surgical system is provided, comprising: a manipulator comprising a robotic arm including a plurality of links and joints and a surgical tool supported and moveable by the robotic arm, wherein the surgical tool is configured to surgically manipulate an anatomy of a patient and is adapted to facilitate collaborative interaction between a surgeon and the manipulator; and a control system coupled to the manipulator and being configured to: acquire robotic data; implement a machine learning model that is configured to receive and process the robotic data to determine an interaction style of the surgeon in collaborating with the manipulator; define a behavior policy based on the determined interaction style, wherein the behavior policy defines a physical feel of the manipulator; and control the manipulator according to the behavior policy.

According to a seventh aspect, a medical system is provided, comprising: a manipulator comprising a robotic arm including a plurality of links and joints and a limb holder supported and moveable by the robotic arm, wherein the limb holder is configured to couple to a limb of a patient and is adapted to facilitate collaborative interaction between the patient and the manipulator; and a control system coupled to the manipulator and being configured to: acquire robotic data; implement a machine learning model that is configured to receive and process the robotic data to determine an interaction style of the patient in collaborating with the manipulator; define a behavior policy based on the determined interaction style, wherein the behavior policy defines a physical feel of the manipulator; and control the manipulator according to the behavior policy.

According to an eighth aspect, a surgical or medical system is provided, comprising: a manipulator comprising a robotic arm including a plurality of links and joints and an end effector supported and moveable by the robotic arm, wherein the end effector is adapted to facilitate collaborative interaction between a human and the manipulator; and a control system coupled to the manipulator and being configured to: control the manipulator according to a behavior policy that defines a physical feel of the manipulator for the human; acquire robotic data; obtain one or more configuration attributes defining a context of the collaborative interaction between the human and the manipulator; implement a machine learning model that is configured to: receive and process the robotic data to determine a target policy; compare the behavior policy and the target policy in view of the one or more configuration attributes; and based on the comparison, determine an interaction style of the human in collaborating with the manipulator; update the behavior policy based on the determined interaction style to customize the physical feel of the manipulator to the determined interaction style; and control the manipulator according to the updated behavior policy.

A computer-implemented method is provided for performing any of the steps implemented by the surgical or medical system or the control system of any one or more of the preceding aspects. A non-transitory computer readable medium or computer program product is provided, comprising instructions, which when executed by one or more processors, are configured to implement the surgical or medical system or the control system of any one or more of the preceding aspects. Also provided is the control system of any one or more of the preceding aspects. Any of the aspects described above can be combined in part, or in whole. Any of the above aspects can be utilized in part, or in whole, with any of the following implementations:

The control system can implement one or more haptic control models. The haptic control models can compute haptic forces that are intended to constrain movement of the end effector in a task space of the manipulator. The behavior policy can be defined based on the one or more haptic control models. The control system can define/refine the behavior policy based on the determined interaction style by being configured to define/refine the one or more haptic control models. The one or more haptic control models can include an active constraint control model. The active constraint control model can compute haptic forces that are intended to constrain specified degrees of freedom of movement of the end effector. The behavior policy can be defined, at least in part, based on the active constraint control model. The control system can define/refine the behavior policy based on the determined interaction style by being configured to define/refine the active constraint control model. The control system can define/refine the active constraint control model by adjusting constraint parameters of any of the specified degrees of freedom, adjusting geometrical parameters of a haptic object utilized by the active constraint control model in computation of the haptic forces that are intended to constrain specified degrees of freedom of movement of the end effector, and/or re-specifying the degrees of freedom of movement that are to be constrained for the end effector. The one or more haptic control models can include a boundary constraint control model. The boundary constraint control model can compute haptic forces that are intended to constrain movement of the end effector relative to a virtual boundary. The behavior policy can be defined, at least in part, based on the boundary constraint control model. The control system can define/refine the behavior policy based on the determined interaction style by being configured to define/refine the boundary constraint control model. The control system can define/refine the boundary constraint control model by adjusting constraint parameters of the virtual boundary, and/or adjusting geometrical parameters of a haptic object utilized by the boundary constraint control model in computation of the haptic forces that are intended to constrain movement of the end effector relative to the virtual boundary. The one or more haptic control models can include a predefined arm feel control model. The predefined arm feel control model can compute haptic forces that are intended to define a baseline physical feel of the manipulator to the human. The behavior policy is defined, at least in part, based on the predefined arm feel control model. The control system can define/refine the behavior policy based on the determined interaction style by being configured to define/refine the predefined arm feel control model. The control system can define/refine the predefined arm feel control model by adjusting a virtual mass of a virtual rigid body model of the end effector, and/or adjust damping parameters of the robotic arm.

The robotic data can include one or more of: prior commanded forces applied to the end effector, prior commanded torques applied to the joints of the robotic arm, prior measured poses of the end effector, and/or prior measured positions of the joints of the robotic arm. The robotic data can include one or more of: prior measured velocities of the end effector, and/or prior measured velocities of the joints of the robotic arm.

The machine learning model can be a model-free adaptive control model, a model predictive control model, a model-free predictive control model, and/or a reinforcement learning model. The machine learning model can determine a target policy based on the robotic data. The machine learning model can compare the behavior policy and the target policy. Based on the comparison, the machine learning model can determine the interaction style of the human in collaborating with the manipulator.

The machine learning model can include a first neural network configured to receive and process the robotic data and determine the target policy based on the robotic data. The first neural network can be part of an evaluator network or critic network. The control system can predict future robotic data and input the future robotic data into the machine learning model. The machine learning model can determine the target policy by optimizing a cost function based on the future robotic data. The control system can obtain desired positions of the end effector and/or the joints of the robotic arm and input the desired positions into the machine learning model. The machine learning model can determine the target policy by optimizing the cost function based on the desired positions. The control system or machine learning model can include a long short-term memory (LSTM) coupled to an input of the machine learning model. The LSTM can include a recurrent neural network (RNN) and is configured to receive values of the robotic data and modify weights of the RNN to learn long-term dependencies among the values and to selectively retain or discard the values.

The machine learning model can include a second neural network. The second neural network can be part of a classifier network or actor network. The second neural network is configured to receive, as an input, the target policy determined by the first neural network. The second neural network can receive the behavior policy as another input. The second neural network can compare the behavior policy and the target policy. Based on the comparison, the second neural network can determine the interaction style of the human in collaborating with the manipulator. The second neural network can receive, as another input, one or more configuration attributes defining a context of the collaborative interaction between the human and the manipulator. The second neural network can facilitate determination of the interaction style of the human in view of the context defined by the one or more configuration attributes. The configuration attributes can specify: environmental attributes, robotic control attributes, application attributes, and/or robotic tool attributes. In the surgical context, configuration attributes can specify: surgical environment attributes, surgical robot control attributes, surgical application attributes, and/or surgical tool attributes. In the medical context, configuration attributes can specify: rehabilitation robot control attributes, rehabilitation application attributes, and/or rehabilitation end effector attributes. The control system can store the updated behavior policy in non-transitory memory. The control system can retrieve the updated behavior policy for a subsequent collaborative interaction between the human and the manipulator and can do so automatically in response to detecting certain conditions or configuration attributes. For a subsequent collaborative interaction between the human and the manipulator, the control system can automatically implement a first updated behavior policy in response to detection of a first condition and automatically implement a second updated behavior policy in response to detection of a second condition.

In the surgical context, the human can be a surgeon. The end effector can be a surgical tool that is configured to surgically manipulate an anatomy of a patient. The surgical tool can be adapted to facilitate collaborative interaction between an arm of the surgeon and the manipulator. The control system can implement the machine learning model to determine the interaction style of the surgeon in collaborating with the manipulator.

In the medical context, the human can be a patient. The end effector can be a limb holder that is configured to couple to a limb of the patient. The limb holder can be adapted to facilitate collaborative interaction between the limb of the patient and the manipulator. The control system can implement the machine learning model to determine the interaction style of the patient in collaborating with the manipulator.

Any of the described implementations can be combined in part, or in whole.

### DESCRIPTION OF THE DRAWINGS

Advantages of the present disclosure will be readily appreciated as the same becomes better understood by reference to the following detailed description when considered in connection with the accompanying drawings.
Figure 1 is a perspective view of a robotic system, according to one implementation.
Figure 2 is a block diagram of an example control system for controlling the robotic system.
Figure 3 is a functional block diagram of modules implemented by the control system, according to one implementation.
Figure 4 illustrates an example output of a boundary generator.
Figure 5 illustrates an example output of a path generator.
Figure 6 is a block diagram of an example control scheme for the manipulator of the robotic system, according to one implementation.
Figure 7 is an example list of equations that can be used by the control system.
Figure 8 is a block diagram of the control system illustrating a machine learning model to determine an interaction style of a human in collaboratively interacting with the manipulator, according to one implementation.
Figure 9 is a block diagram of a robotic data unit that can pre-process robotic data to be utilized by the machine learning model, according to one implementation.
Figure 10 is a block diagram of one implementation of the machine learning model to determine the interaction style of the human in collaboratively interacting with the manipulator.
Figure 11 is a diagram illustrating example interaction style classifications that can be determined by the machine learning model.
Figure 12 is a block diagram of other implementations of the machine learning model to determine the interaction style, wherein the machine learning model employs a model-free adaptive control (MFAC) or model-predictive control (MPC) approach.
Figure 13 is a block diagram of another implementation of the machine learning model to determine the interaction style, wherein the machine learning model employs a reinforcement learning model approach.

### DETAILED DESCRIPTION

### I. Example System Overview

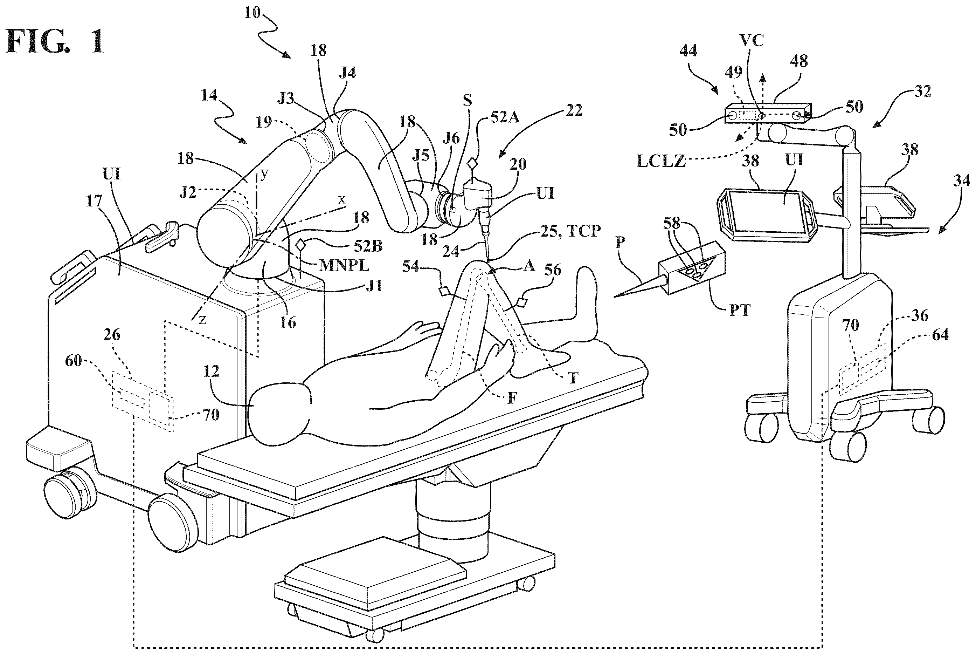

Referring to Figure 1, a surgical/medical system 10 is illustrated. As will be described below, the system 10 can be used for surgical purposes or for patient rehabilitation purposes in collaboration with a surgeon or patient, respectively.

For surgery, the system 10 is useful for treating a surgical site or anatomical volume (A) of a patient 12, such as treating bone or soft tissue. In Figure 1, the patient 12 is undergoing a surgical procedure. The anatomy in Figure 1 includes a femur F and a tibia T of the patient 12. The surgical procedure may involve tissue removal or other forms of treatment. Treatment may include cutting, coagulating, lesioning the tissue, other in-situ tissue treatments, or the like. In some examples, the surgical procedure involves partial or total knee or hip replacement surgery, shoulder replacement surgery, spine surgery, or ankle surgery. In some examples, the system 10 is designed to cut away material to be replaced by surgical implants, such as hip and knee implants, including unicompartmental, bicompartmental, multicompartmental, or total knee implants. Some of these types of implants are shown in U.S. Patent Application Publication No. 2012/0330429, entitled, "Prosthetic Implant and Method of Implantation," the disclosure of which is hereby incorporated by reference. The system 10 and techniques disclosed herein may be utilized to perform other procedures, surgical or non-surgical, or may be utilized in industrial applications or other applications where robotic systems are utilized.

The surgical/medical system 10 includes a manipulator 14 or robotic manipulator. The manipulator 14 can be a collaborative surgical robot or a collaborative patient rehabilitation robot. Collaborative means that the surgeon/patient directly interacts with the manipulator 14 by applying forces to the manipulator 14.

The manipulator 14 has a base 16 and plurality of links 18 and joints (J). A manipulator cart 17 supports the manipulator 14 such that the manipulator 14 is fixed to the manipulator cart 17. The links 18 collectively form one or more arms of the manipulator 14. The manipulator 14 may have a serial arm configuration (as shown in Figure 1), a parallel arm configuration, or any other suitable manipulator configuration. In other examples, more than one manipulator 14 may be utilized in a multiple arm configuration. In other configurations, the manipulator 14 can include a partially or entirely adjustable arm (AA), with passive joints that manually adjustable. The joints of the adjustable arm (AA) optionally may be locked in position by the user. In other instances, some or all the joints of the adjustable arm (AA) may be actively driven using joint motors.

The manipulator 14 can include a passive (manually articulated) joint, such as planarly extending joint. For instance, the passive joint may by the distal most joint attached to the robotic arm comprising a plurality of active joints. The planarly extending joint can support a tool such as a saw blade to allow the user to manually move the saw blade along a plane. In this case, the tool is mechanically constrained to the plane by the mechanical constraints of the passive joint, while the pose of the plane is actively constrained by the active joints (J) of the robotic arm. In other instances, one or more joints (J) of the manipulator 14 could be controlled to constrain the tool in certain degrees of freedom, while allowing the tool free passive motion in other degrees of freedom or manners, such as a rotation about a point or a selected linear motion.

The manipulator 14 can exhibit kinematic redundancy wherein the tool positions are defined by up to 6DOF, but the robotic arm may operate in more than 6DOF. With such redundancy, the manipulator 14 can utilize null-space controls whereby the joints (J) of the manipulator 14 can change positions while maintaining the pose of the end effector 22. Null-space controls can also be used to avoid singularities.

In the example shown in Figure 1, the manipulator 14 comprises a plurality of joints (J) and a plurality of joint encoders 19 located at the joints (J) for determining position data of the joints (J). For simplicity, only one joint encoder 19 is illustrated in Figure 1, although other joint encoders 19 may be similarly illustrated. The manipulator 14 according to one example has six joints J1-J6 implementing at least six-degrees of freedom (DOF) for the manipulator 14. However, the manipulator 14 may have any number of degrees of freedom and may have any suitable number of joints J and may have redundant joints.

The manipulator 14 need not require joint encoders 19 but may alternatively, or additionally, utilize motor encoders present on motors at each joint J. Also, the manipulator 14 need not require rotary joints, but may alternatively, or additionally, utilize one or more prismatic joints. Any suitable combination of joint types is contemplated.

The base 16 of the manipulator 14 is a portion of the manipulator 14 that provides a fixed reference coordinate system for other components of the manipulator 14 or the system 10 in general. The origin of a manipulator coordinate system MNPL is defined at the fixed reference of the base 16. The base 16 may be defined with respect to any suitable portion of the manipulator 14, such as one or more of the links 18. Alternatively, or additionally, the base 16 may be defined with respect to the manipulator cart 17, such as where the manipulator 14 is physically attached to the manipulator cart 17. In one example, the base 16 is defined at an intersection of the axes of joints J1 and J2. Thus, although joints J1 and J2 are moving components in reality, the intersection of the axes of joints J1 and J2 is nevertheless a virtual fixed reference pose, which provides both a fixed position and orientation reference and which does not move relative to the manipulator 14 and/or manipulator cart 17.

In other examples, the manipulator 14 can be a hand-held manipulator where the base 16 is a base portion of a tool (e.g., a portion held free hand by the user), and the tool tip is movable relative to the base portion. The base portion has a reference coordinate system that is tracked, and the tool tip has a tool tip coordinate system that is computed relative to the reference coordinate system (e.g., via motor and/or joint encoders and forward kinematic calculations). Movement of the tool tip can be controlled to follow the path since its pose relative to the path can be determined. The hand-held manipulator 14 can be like that described and shown in US20230255701, entitled "Systems and Methods for Guiding Movement of a Handheld Medical Robotic Instrument", the entire disclosure of which is hereby incorporated by reference.

The manipulator 14 and/or manipulator cart 17 house a manipulator controller 26, or other type of control unit. The manipulator controller 26 may comprise one or more computers, or any other suitable form of controller that directs the motion of the manipulator 14. The manipulator controller 26 may have a central processing unit (CPU), graphics processing unit (GPU) and/or other processors, memory, and storage. The manipulator controller 26 is loaded with software as described below. The processors could include one or more processors to control operation of the manipulator 14. The processors can be any type of microprocessor, multi-processor, and/or multi-core processing system. The manipulator controller 26 may additionally, or alternatively, comprise one or more microcontrollers, field programmable gate arrays, systems on a chip, discrete circuitry, and/or other suitable hardware, software, or firmware that is capable of carrying out the functions described herein. The term processor is not intended to limit any embodiment to a single processor. The manipulator 14 may also comprise a user interface UI with one or more displays and/or input devices (e.g., push buttons, keyboard, mouse, microphone (voice-activation), gesture control devices, touchscreens, etc.).

When the manipulator 14 is a surgical robot, a tool 20 couples to the manipulator 14 and is movable relative to the base 16 to interact with the anatomy in certain modes. The tool 20 is a physical and surgical tool and is, or forms part of, the end effector 22 supported by the manipulator 14 in certain implementations. The tool 20 or end effector 22 may be grasped by the user. One possible arrangement of the manipulator 14 and the tool 20 is described in U.S. Patent No. 9,119,655, entitled, "Surgical Manipulator Capable of Controlling a Surgical Instrument in Multiple Modes," the disclosure of which is hereby incorporated by reference. The manipulator 14 and the end effector 22 may be arranged in alternative configurations. The end effector 22 can be like that shown in U.S. Patent Application Publication No. 2014/0276949, filed on March 15, 2014, entitled, "End Effector of a Surgical Robotic Manipulator," hereby incorporated by reference.

The tool 20 can include an energy applicator 24 designed to contact and remove the tissue of the patient 12 at the surgical site. In one example, the energy applicator 24 is a bur 25. The bur 25 may be substantially spherical and comprise a spherical center, radius (r) and diameter. Alternatively, the energy applicator 24 may be a drill bit, a saw blade, an ultrasonic vibrating tip, or the like. The tool 20 and/or energy applicator 24 may comprise any geometric feature, e.g., perimeter, circumference, radius, diameter, width, length, volume, area, surface/plane, range of motion envelope (along any one or more axes), etc. The geometric feature may be considered to determine how to locate the tool 20 relative to the tissue at the surgical site to perform the desired treatment. In some of the embodiments described herein, a spherical bur having a tool center point (TCP) will be described for convenience and ease of illustration but is not intended to limit the tool 20 to any particular form. In other examples, the tool 20 does not include an energy applicator 24. For example, the tool 20 can be a slotted cut guide for a saw, a guide tube for receiving another tool, or the like.

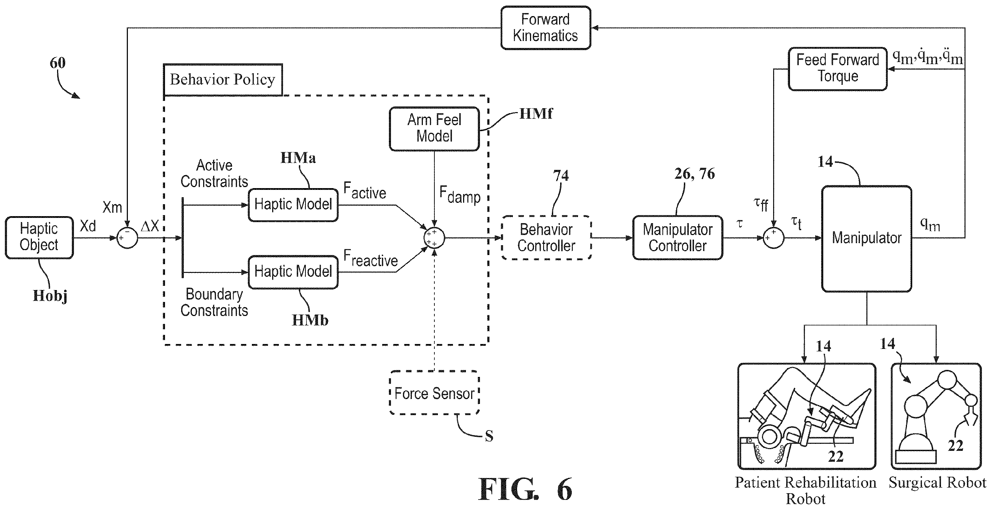

When the manipulator 14 is a patient rehabilitation robot (as shown in the example of FIG. 6), the manipulator 14 is part of robotic-assisted rehabilitation platform to medically assist patients. In such situations, the patient directly interacts with the manipulator 14 (or fixture attached to the manipulator) by applying a force to move the manipulator 14 and perform guided movements or exercises. The rehabilitation robot can be used for a variety of purposes, such as for providing physical therapy, improving patient mobility, improving patient functional strength, and the like. Any suitable end effector 22 (tool, holder, or device) may be attached to the manipulator 14 to facilitate collaborative interaction with the patient. For example, the end effector 22 may be a limb holder, such as for holding one or more legs of the patient (as shown), one or more arms of the patient, the patient hand(s), the patient foot/feet, or the like. The manipulator 14 may have any configuration described above, such as a serial arm configuration, or the like. The end effector 22 of the rehabilitation robot may include a handle and/or user interface (buttons) to enable a staff member to also collaborate with the robot. In some cases, the manipulator 14 can have an exoskeleton configuration or can support major portions of the patient's body, such as the upper body, lower body, or the like.

The end effector 22 may comprise a tool controller to control operation of the end effector 22, such as to control power to the tool (e.g., to a rotary motor of the tool 20), control movement of the tool 20, control irrigation/aspiration of the tool 20, and/or the like. The tool controller may be in communication with the manipulator controller 26 or other components. The end effector 22 may also comprise a user interface UI with one or more displays and/or input devices (e.g., push buttons, keyboard, mouse, microphone (voice-activation), gesture control devices, touchscreens, etc.). For example, one of the user input devices on the user interface UI of the end effector 22 may be a tool input (e.g., switch or other form of user input device) that has first and second input states (see FIG. 1). The tool input can be actuated (e.g., pressed and held) by the user to be placed in the first input state and can be released to be placed in the second input state. The end effector 22 may have a grip on which the tool input is located. In some versions, the tool input is a presence detector that detects the presence of a hand of the user, such as a momentary contact switch that switches between on/off states, a capacitive sensor, an optical sensor, or the like. The tool input is thus configured such that the first input state indicates that a user is actively engaging the end effector 22 and the second input state indicates that the user has released the end effector 22. The tool input may be a continuous activation device, i.e., inputs that must be continually actuated to allow motion of the end effector 22 in the manual mode or the semi-autonomous mode, depending on which user input is actuated. For example, while the user is continually actuating the tool input, and the manual mode is enabled, the manipulator 14 will move in response to the input forces and torques applied by the user and the control system 60 will enforce the virtual boundary 71 to protect the patient anatomy. When the tool input is released, input from the force/torque sensor S may be disabled such that the manipulator 14 no longer responds to the forces and torques applied by the user to the end effector 22.

The manipulator controller 26 controls a state (position and/or orientation) of the tool 20 (e.g., the TCP) with respect to a coordinate system, such as the manipulator coordinate system MNPL. The manipulator controller 26 can control (linear or angular) velocity, acceleration, or other derivatives of motion of the tool 20. The tool center point (TCP), in one example, is a predetermined reference point defined at the energy applicator 24. The TCP has a known, or able to be calculated (i.e., not necessarily static), pose relative to other coordinate systems. The geometry of the energy applicator 24 is known in or defined relative to a TCP coordinate system. The TCP may be located at the spherical center of the bur 25 of the tool 20 such that only one point is tracked. The TCP may be defined in several ways depending on the configuration of the energy applicator 24. The manipulator 14 could employ the joint/motor encoders, or any other non-encoder position sensing method, to enable a pose of the TCP to be determined. The manipulator 14 may use joint measurements to determine TCP pose and/or could employ techniques to measure TCP pose directly. The control of the tool 20 is not limited to a center point. For example, any suitable primitives, meshes, etc., can be utilized to represent the tool 20.

The surgical/medical system 10 may include a navigation system 32. One example of the navigation system 32 is described in U.S. Patent No. 9,008,757, filed on September 24, 2013, entitled, "Navigation System Including Optical and Non-Optical Sensors," hereby incorporated by reference. The navigation system 32 tracks movement of various objects. Such objects include, for example, the manipulator 14, the tool 20/end effector 22 and the anatomy, e.g., femur F and tibia T, or patient limbs. The navigation system 32 tracks these objects to gather state information of each object with respect to a (navigation) localizer coordinate system LCLZ. Coordinates in the localizer coordinate system LCLZ may be transformed to the manipulator coordinate system MNPL, and/or vice-versa, using transformations.

The navigation system 32 includes a cart assembly 34 that houses a navigation controller 36, and/or other types of control units. A navigation user interface UI is in operative communication with the navigation controller 36. The navigation user interface includes one or more displays 38. The navigation system 32 can display a graphical representation of the relative states of the tracked objects to the user using the one or more displays 38. The navigation user interface UI further comprises one or more input devices to input information into the navigation controller 36 or otherwise to select/control certain aspects of the navigation controller 36. Such input devices include interactive touchscreen displays. However, the input devices may include any one or more of push buttons, a keyboard, a mouse, a microphone (voice-activation), gesture control devices, and the like.

The navigation system 32 also includes a localizer 44 coupled to the navigation controller 36. In one example, the localizer 44 is an optical localizer and includes a camera unit 46. The camera unit 46 has an outer casing 48 that houses one or more optical sensors 50. The localizer 44 may comprise its own localizer controller 49 and may further comprise a video camera VC.

The navigation system 32 includes one or more trackers. In one example, the trackers include a pointer tracker PT, one or more manipulator trackers 52A, 52B, a first patient tracker 54, and a second patient tracker 56. In the illustrated example of Figure 1, the manipulator tracker is rigidly attached to the end effector 22 (i.e., tracker 52A), the first patient tracker 54 is coupled to the femur F of the patient 12, and the second patient tracker 56 is coupled to the tibia T of the patient 12. The patient trackers 54, 56 may be firmly affixed to sections of bone or could be coupled to the exterior of the leg. The pointer tracker PT is firmly affixed to a pointer or probe P utilized for registering the anatomy to the localizer coordinate system LCLZ. The manipulator tracker 52A, 52B may be affixed to any suitable component of the manipulator 14, in addition to, or other than the end effector 22, such as the base 16 (i.e., tracker 52B), or any one or more links 18 of the manipulator 14. The trackers 52A, 52B, 54, 56, PT may be fixed to their respective components in any suitable manner. For example, the trackers may be rigidly fixed, flexibly connected (optical fiber), or not physically connected at all (ultrasound), as long as there is a suitable (supplemental) way to determine the relationship (measurement) of that respective tracker to the object with which it is associated.

Any one or more of the trackers may include active markers 58. The active markers 58 may include light emitting diodes (LEDs). Alternatively, the trackers 52A, 52B, 54, 56, PT may have passive markers, such as reflectors, which reflect light emitted from the camera unit 46. Other suitable markers not specifically described herein may be utilized.

The localizer 44 tracks the trackers 52A, 52B, 54, 56, PT to determine a state of each of the trackers 52A, 52B, 54, 56, PT, which correspond respectively to the state of the object respectively attached thereto. The localizer 44 may perform known triangulation techniques to determine the states of the trackers 52, 54, 56, PT, and associated objects. The localizer 44 provides the state of the trackers 52A, 52B, 54, 56, PT to the navigation controller 36. In one example, the navigation controller 36 determines and communicates the state the trackers 52A, 52B, 54, 56, PT to the manipulator controller 26. As used herein, the state of an object includes, but is not limited to, data that defines the position and/or orientation of the tracked object or equivalents/derivatives of the position and/or orientation. For example, the state may be a pose of the object, and may include linear velocity data, and/or angular velocity data, and the like.

The navigation controller 36 may comprise one or more computers, or any other suitable form of controller. Navigation controller 36 may have a central processing unit (CPU), graphics processing unit (GPU) and/or other processors, non-transitory memory, and storage. The processors can be any type of processor, microprocessor, or multi-processor system. The navigation controller 36 is loaded with software. The software, for example, converts the signals received from the localizer 44 into data representative of the position and orientation of the objects being tracked. The navigation controller 36 may additionally, or alternatively, comprise one or more microcontrollers, field programmable gate arrays, systems on a chip, discrete circuitry, and/or other suitable hardware, software, or firmware that can conduct the functions described herein. The term processor is not intended to limit any embodiment to a single processor.

Although one example of the navigation system 32 is shown that employs triangulation techniques to determine object states, the navigation system 32 may have any other suitable configuration for tracking the manipulator 14, end effector 22, and/or the patient 12.

In another example, the navigation system 32 and/or localizer 44 are ultrasound-based. For example, the navigation system 32 may comprise an ultrasound imaging device coupled to the navigation controller 36. The ultrasound imaging device images any of the aforementioned objects, e.g., the manipulator 14, the end effector 22, and/or the patient 12, and generates state signals to the navigation controller 36 based on the ultrasound images. The ultrasound images may be 2-D, 3-D, or a combination of both. The navigation controller 36 may process the images in near real-time to determine states of the objects. The ultrasound imaging device may have any suitable configuration and may be different than the camera unit 46 as shown in Figure 1.

In another example, the navigation system 32 and/or localizer 44 are radio frequency (RF)-based. For example, the navigation system 32 may comprise an RF transceiver coupled to the navigation controller 36. The manipulator 14, the end effector 22, and/or the patient 12 may comprise RF emitters or transponders attached thereto. The RF emitters or transponders may be passive or actively energized. The RF transceiver transmits an RF tracking signal and generates state signals to the navigation controller 36 based on RF signals received from the RF emitters. The navigation controller 36 may analyze the received RF signals to associate relative states thereto. The RF signals may be of any suitable frequency. The RF transceiver may be positioned at any suitable location to track the objects using RF signals effectively. Furthermore, the RF emitters or transponders may have any suitable structural configuration that may be much different than the trackers 52A, 52B, 54, 56, PT shown in Figure 1.

In yet another example, the navigation system 32 and/or localizer 44 are electromagnetically based. For example, the navigation system 32 may comprise an EM transceiver coupled to the navigation controller 36. The manipulator 14, the end effector 22, and/or the patient 12 may comprise EM components attached thereto, such as any suitable magnetic tracker, electro-magnetic tracker, inductive tracker, or the like. The trackers may be passive or actively energized. The EM transceiver generates an EM field and generates state signals to the navigation controller 36 based upon EM signals received from the trackers. The navigation controller 36 may analyze the received EM signals to associate relative states thereto. Again, such navigation system examples may have structural configurations that are different than the navigation system 32 configuration shown in Figure 1.

The navigation system 32 may have any other suitable components or structure not specifically recited herein. Furthermore, any of the techniques, methods, and/or components described above with respect to the navigation system 32 shown may be implemented or provided for any of the other examples of the navigation system 32 described herein. For example, the navigation system 32 may utilize solely inertial tracking or any combination of tracking techniques, and may additionally or alternatively comprise, fiber optic-based tracking, machine-vision tracking, and the like.

The pointer P may be hand-held or may optionally be coupled to the adjustable arm (AA), as shown in FIG. 1. The pointer P may include a force/torque sensor S' that is configured to measure forces/torques applied to the pointer tip. The pointer P may also include a controller, e.g., housed within the pointer body to process measurements from the force/torque sensor S'. Measurements from the pointer P may be communicated to the control system 60 using a wired or wireless connection.

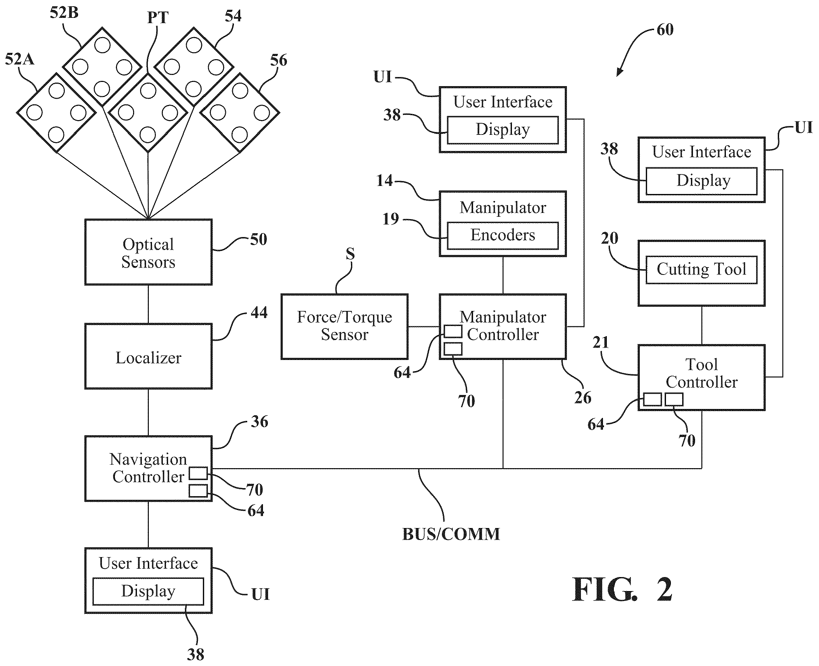

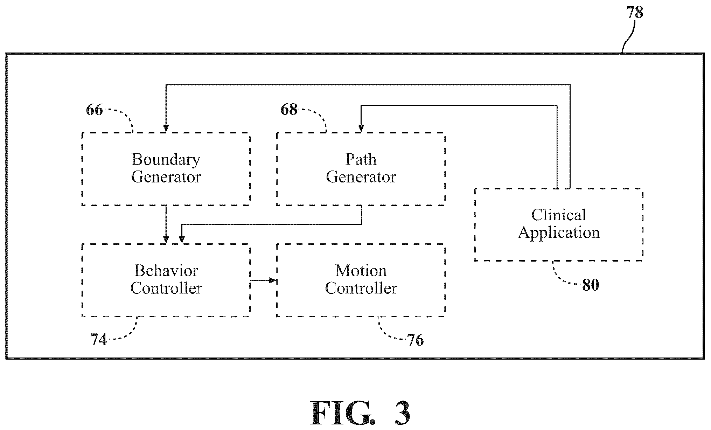

Referring to Figure 2, the system 10 includes the control system 60 that comprises, among other components, the manipulator controller 26, the navigation controller 36, and the tool controller 21. The control system 60 further includes one or more software programs and software modules shown in Figure 3. The software modules may be part of the program or programs that operate on the manipulator controller 26, navigation controller 36, tool controller 21, or any combination thereof, to process data to assist with control of the system 10. The software programs and/or modules include computer readable instructions stored in non-transitory memory 64 on the manipulator controller 26, navigation controller 36, tool controller 21, or a combination thereof, to be executed by one or more processors 70 of the controllers 21, 26, 36. The memory 64 may be any suitable configuration of memory, such as RAM, non-volatile memory, etc., and may be implemented locally or from a remote database. Additionally, software modules for prompting and/or communicating with the user may form part of the program or programs and may include instructions stored in memory 64 on the manipulator controller 26, navigation controller 36, tool controller 21, or any combination thereof. The user may interact with any of the input devices of the navigation user interface UI or other user interface UI to communicate with the software modules. The user interface software may run on a separate device from the manipulator controller 26, navigation controller 36, and/or tool controller 21.

The control system 60 may comprise any suitable configuration of input, output, and processing devices suitable for conducting the functions and methods described herein. The control system 60 may comprise the manipulator controller 26, the navigation controller 36, or the tool controller 21, or any combination thereof, or may comprise only one of these controllers. These controllers may communicate via a wired bus or communication network as shown in Figure 2, via wireless communication, or otherwise. The control system 60 may also be referred to as a controller. The control system 60 may comprise one or more microcontrollers, field programmable gate arrays, systems on a chip, discrete circuitry, sensors, displays, user interfaces, indicators, and/or other suitable hardware, software, or firmware that can perform the functions described herein.

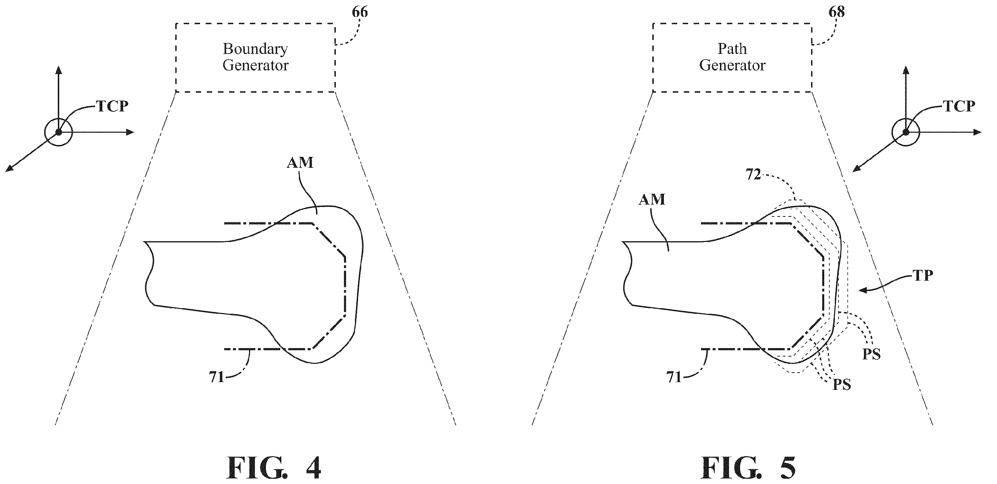

Referring to Figure 3, the software employed by the control system 60 includes a boundary generator 66. As shown in Figure 4, the boundary generator 66 is a software program or module that generates a virtual boundary 71 for constraining movement and/or operation of the end effector 22. The virtual boundary 71 may be one-dimensional, two-dimensional, three-dimensional, and may comprise a point, line, axis, trajectory, plane, or other shapes, including complex geometric shapes. In some embodiments, the virtual boundary 71 is a surface defined by a triangle mesh. Such virtual boundaries 71 may also be referred to as virtual objects. The virtual boundaries 71 may be defined with respect to an anatomical model AM, such as a 3-D bone model. In the example of Figure 4, the virtual boundaries 71 are planar boundaries to delineate five planes for a total knee implant, and are associated with a 3-D model of the head of the femur F. The anatomical model AM is registered to the one or more patient trackers 54, 56 such that the virtual boundaries 71 become associated with the anatomical model AM. The virtual boundaries 71 may be implant-specific, e.g., defined based on a size, shape, volume, etc. of an implant and/or patient-specific, e.g., defined based on the patient's anatomy. The virtual boundaries 71 may be boundaries that are created pre-operatively, intra-operatively, or combinations thereof. In other words, the virtual boundaries 71 may be defined before the procedure begins, during the procedure (including during tissue removal), or combinations thereof. In any case, the control system 60 obtains the virtual boundaries 71 by storing/retrieving the virtual boundaries 71 in/from memory, obtaining the virtual boundaries 71 from memory, creating the virtual boundaries 71 pre-operatively, creating the virtual boundaries 71 intra-operatively, or the like.

The manipulator controller 26 and/or the navigation controller 36 track the state of the end effector 22 relative to the virtual boundaries 71. In one example, the state of the TCP is measured relative to the virtual boundaries 71 for purposes of determining haptic forces to be applied to a virtual rigid body model via a virtual simulation 88 so that the end effector 22 remains in a desired positional relationship to the virtual boundaries 71 (e.g., not moved beyond them). The results of the virtual simulation 88 are commanded to the manipulator 14. The control system 60 controls/positions the manipulator 14 in a manner that emulates the way a physical handpiece would respond in the presence of physical boundaries/barriers. The boundary generator 66 may be implemented on the manipulator controller 26. Alternatively, the boundary generator 66 may be implemented on other components, such as the navigation controller 36.

Referring to Figures 3 and 5, a path generator 68 is another software program or module run by the control system 60. In one example, the path generator 68 is run by the manipulator controller 26. The path generator 68 generates a tool path TP for the tool 20 to traverse. The tool path TP may comprise a plurality of path segments PS, or may comprise a single path segment PS. The path segments PS may be straight segments, curved segments, combinations thereof, or the like. The tool path TP may be defined with respect to the manipulator 14 coordinate system MNPL, localizer coordinate system LCLZ, coordinate system of the tool 20, coordinate system of the anatomy, or any combination thereof. The tool path TP can be virtually attached to the coordinate system of the respective object such that if the object were to move, the tool path TP will correspondingly move. The tool path TP may be implant-specific, e.g., defined based on a size, shape, volume, etc. of an implant and/or patient-specific, e.g., defined based on the patient's anatomy. The tool path TP can be associated with a virtual model of the anatomy and the virtual model and tool path can be registered to the anatomy using the navigation system 32. The control system 60 can generate or obtain the tool path TP by storing/retrieving the tool path TP in/from memory, creating the tool path TP pre-operatively, creating the tool path TP intra-operatively, or the like. The tool path TP may have any 3D shape, or combinations of shapes, such as circular, helical/corkscrew, linear, curvilinear, combinations thereof, and the like.

In one implementation, the tool path TP is defined as a guidance or alignment path. In one example, the tool path TP is for guiding the tool 20 to move to a location that positions the tool 20 for a start of the surgical procedure, or step. For instance, if the tool 20 is a saw blade, the tool path TP may be configured to guide the saw blade to align to a cut plane associated with the anatomy. If the tool 20 is a cutting bur, the tool path TP may be configured to guide the cutting bur to a starting point in preparation for automated cutting. A lead-in path could be virtually connected from the starting point to another cutting path for removal of tissue. The tool path TP may also enable the tool 20 to move along a predefined path of motion for purposes of registering components of the manipulator 14 to the navigation system 32. The tool path TP can be registered to the anatomy using the navigation system 32 such that the tool path TP is virtually fixed to the anatomy. This way, the tool path TP location in space will automatically be updated to account for any movement of the anatomy.

In another implementation, as shown in FIG. 5, the tool path TP is defined as a tissue removal path. One example of the tissue removal path described herein comprises a milling path 72. The term "milling path" refers to the path of the tool 20 in the vicinity of the target site for milling the anatomy and is not intended to require that the tool 20 be operably milling the anatomy throughout the entire duration of the path. For instance, as will be understood in further detail below, the milling path 72 may comprise sections or segments where the tool 20 transitions from one location to another without milling. Additionally, other forms of tissue removal along the milling path 72 may be employed, such as tissue ablation, and the like. The milling path 72 may be a predefined path that is created pre-operatively, intra-operatively, or combinations thereof. In other words, the milling path 72 may be defined before the surgical procedure begins, during the surgical procedure (including during tissue removal), or combinations thereof.

One example of a system and method for generating the virtual boundaries 71 and/or the milling path 72 is described in U.S. Patent No. 9,119,655, entitled, "Surgical Manipulator Capable of Controlling a Surgical Instrument in Multiple Modes," the disclosure of which is hereby incorporated by reference. In some examples, the virtual boundaries 71 and/or tool paths TP may be generated offline rather than on the manipulator controller 26 or navigation controller 36. Thereafter, the virtual boundaries 71 and/or tool paths TP may be utilized at runtime by the manipulator controller 26.

Referring to Figure 3, two additional software programs or modules run on the manipulator controller 26 and/or the navigation controller 36. One software module is a behavior controller 74. Behavior controller 74 can compute data that indicates the next commanded pose and/or orientation (e.g., pose) for the end effector 22. In some cases, only the position of the TCP is output from the behavior controller 74, while in other cases, the position and orientation of the end effector 22 is output. Output from the boundary generator 66, the path generator 68, and a force/torque sensor S may feed as inputs into the behavior controller 74 to determine the next commanded pose and/or orientation for the end effector 22. The behavior controller 74 may process these inputs, along with one or more virtual constraints described further below, to determine the commanded pose. The behavior controller 74 can be implemented in an admittance control mode, wherein the robotic system proactively generates the commanded position based on an output of a virtual rigid body simulation.

The second software module can include a motion controller 76. One aspect of motion control is the control of the manipulator 14. The motion controller 76 receives data defining the next commanded pose from the behavior controller 74. Based on these data, the motion controller 76 determines the next position of the joint angles of the joints (J) of the manipulator 14 (e.g., via inverse kinematics and Jacobian calculators) so that the manipulator 14 is able to position the end effector 22 as commanded by the behavior controller 74, e.g., at the commanded pose. In other words, the motion controller 76 processes the commanded pose, which may be defined in Cartesian space, into joint angles of the manipulator 14, so that the manipulator controller 26 can command the joint motors accordingly, to move the joints (J) of the manipulator 14 to commanded joint angles corresponding to the commanded pose of the end effector 22. In one version, the motion controller 76 regulates the joint angle of each joint J and continually adjusts the torque that each joint motor outputs to, as closely as possible, ensure that the joint motor drives the associated joint J to the commanded joint angle.

The boundary generator 66, path generator 68, behavior controller 74, and motion controller 76 may be sub-sets of a software program 78. Alternatively, each may be software programs that operate separately and/or independently in any combination thereof. The term "software program" is used herein to describe the computer-executable instructions that are configured to perform the various capabilities of the technical solutions described. For simplicity, the term "software program" is intended to encompass, at least, any one or more of the boundary generator 66, path generator 68, behavior controller 74, and/or motion controller 76. The software program 78 can be implemented on the manipulator controller 26, navigation controller 36, or any combination thereof, or may be implemented in any suitable manner by the control system 60.

A clinical application 80 may be provided to manage user interaction. The clinical application 80 handles many aspects of user interaction and coordinates the surgical/medical workflow, including pre-operative planning, implant placement, registration, bone preparation visualization, and post-operative evaluation of implant fit, etc. The clinical application 80 is configured to output to the displays 38. The clinical application 80 may run on its own separate processor or may run alongside the navigation controller 36. In one example, the clinical application 80 interfaces with the boundary generator 66 and/or path generator 68 after implant placement is set by the user, and then sends the virtual boundary 71 and/or tool path TP returned by the boundary generator 66 and/or path generator 68 to the manipulator controller 26 for execution. Manipulator controller 26 executes the tool path TP as described herein. The manipulator controller 26 may additionally create certain segments (e.g., lead-in segments) when starting or resuming machining to smoothly get back to the generated tool path TP. The manipulator controller 26 may also process the virtual boundaries 71 to generate corresponding virtual constraints as described further below.

The system 10 may operate in a manual mode, such as described in U.S. Patent No. 9,119,655, incorporated herein by reference. Here, the user manually directs, and the manipulator 14 executes movement of the end effector 22. The user physically contacts the end effector 22 to cause movement in the manual mode. In one version, the manipulator 14 monitors forces and torques placed on the end effector 22 by the user to position the end effector 22. For example, the manipulator 14 may comprise the force/torque sensor S that detects the forces and torques applied by the user and generates corresponding input utilized by the control system 60 (e.g., one or more corresponding input/output signals). In some implementations, the user may be required to continually grasp a trigger or switch on the end effector 22 to enable the force/torque sensor S that detects the forces and torques applied by the user.

The force/torque sensor S may comprise a 6-DOF force/torque transducer. The manipulator controller 26 and/or the navigation controller 36 receives the input (e.g., signals) from the force/torque sensor S. In response to the user-applied forces and torques, the manipulator 14 moves the end effector 22 in a manner that emulates the movement that would have occurred based on the forces and torques applied by the user. Movement of the end effector 22 in the manual mode may also be constrained in relation to the virtual boundaries 71 generated by the boundary generator 66. In some versions, measurements taken by the force/torque sensor S are transformed from a force/torque coordinate system FT of the force/torque sensor S to another coordinate system, such as a virtual mass coordinate system VM in which the virtual simulation 88 is carried out on the virtual rigid body model of the end effector 22 so that the forces and torques can be virtually applied to the virtual rigid body in the virtual simulation 88 to ultimately determine how those forces and torques (among other inputs) would affect movement of the virtual rigid body, as described below.

The system 10 may also operate in a semi-autonomous or automated mode in which the manipulator 14 moves the end effector 22 along the milling path 72 (e.g., the active joints (J) of the manipulator 14 operate to move the end effector 22 without requiring force/torque on the end effector 22 from the user). An example of operation in the automated mode is also described in U.S. Patent No. 9,119,655, incorporated herein by reference. In some embodiments, when the manipulator 14 operates in the automated mode, the manipulator 14 can move the end effector 22 free of user applied forces. In other words, the user does not need to physically contact the end effector 22 to move the end effector 22. Instead, the user may use some form of remote control to control starting and stopping of movement. For example, the user may hold down a button of the remote control to start movement of the end effector 22 and release the button to stop movement of the end effector 22.

The system 10 may also operate in a guided-manual mode, as described in U.S Patent Application Publication No. US 2020/0281676 A1, entitled "Systems and Methods for Controlling Movement of a Surgical Tool Along a Predefined Path", the contents of which are hereby incorporated by reference in their entirety. In the guided-manual mode, the user applies forces/torques to the force/torque sensor S and the applied forces/torques are utilized to determine how far to advance the end effector 22 along the tool path TP. In the guided-manual mode, the end effector 22 is constrained to the tool path TP in 2DOF normal to the tool path, but unconstrained in 1DOF tangential to the tool path TP. In effect, this enables the end effector 22 to freely move along the tool path TP based on manual input, but the constraints guide the user by restricting the manual movement of the end effector 22 to be along the tool path.

The techniques described herein can utilize constraint equations and data, forward dynamics algorithms, rigid body calculations, constraint force calculations, and virtual simulations like those described in U.S Patent Application Publication No. US 2020/0281676 A1, entitled "Systems and Methods for Controlling Movement of a Surgical Tool Along a Predefined Path", the contents of which are hereby incorporated by reference in their entirety.

### A. Example Manipulator Control Scheme

Referring to FIG 6, described in this section is an example control scheme implemented by the control system 60 for controlling the manipulator 14 using haptic forces and joint torques. In one implementation, the control system 60 is configured to implement constraints on the manipulator 14 or the end effector 22 pursuant to predefined virtual fixtures or haptic objects (Hobj). Although the robot control problem varies for each type of surgical procedure or tool, these constraints can be divided into two subspaces i.e., active constraints and boundary constraints. The active and boundary constraints impose "task space" constraints on movement of the end effector 22. The task space is the Cartesian space defined by the task the manipulator 14 is performing. The task space can be defined by the set of all possible poses of the end effector 22 for the given task. The dimensions of the task space will depend on the surgical procedure, step of the procedure, type, or end effector 22, or the like. For example, tasks in robotics-assisted arthroplasty require up to six DOF of manipulator control depending on the type of the resection and the end effector 22 used for that resection. As an example, total knee arthroplasty (TKA) saw cutting can be fully constrained task which requires the saw blade to be fully controlled in all six DOFs. Screw/post placement task in shoulder and spine surgery may require a burring attachment with robot control in five DOF (roll motion control is not required during burring). The task space for robotic surgery may be defined by a coordinate system of the implant, patient, or robot, or combinations thereof.

Active constraints attempt to actively virtually constrain specified DOF(s) of the end effector 22. Active constraints provide the surgeon with resistance or guidance of the end effector 22 by actively restricting a surgeon's movement of the surgical tool in a specified manner. For example, active constraints can constrain certain DOF of the surgical tool so that the surgical tool will feel stiff if the surgeon attempts to move the tool in one of the constrained DOF. Such active constraints can be imposed in any of the described operating modes of the manipulator 14, i.e., manual mode, automated mode, guided-manual mode, etc. It is not necessary that the surgeon interact with the end effector 22 to impose the active constraints.

Boundary constraints are used to provide a boundary on movement of the end effector 22, e.g., to keep the surgical tool in a zone or keep the surgical tool out of a zone. The boundary constraints are the virtual boundaries 71 that are generated by the boundary generator 66, which have been described above. For the boundary constraints, a reaction force is generated in response to contact or potential contact of the end effector 22 to the virtual boundary 71. Such boundary constraints can be imposed in any of the described operating modes of the manipulator 14, i.e., manual mode, semi-autonomous mode, guided-manual mode, etc.

In some cases, the boundary and active constraints may be activated only under certain conditions. For example, one condition is when the end effector 22 is located proximity to the surgical site or when the tool enters a "zone" such as a resection zone, cutting zone or any zone where the surgical or medical procedure is to be carried out.

As shown in FIG. 6, the control system 60 may be configured to implement several haptic control models. Among these models is a haptic control model (HMb) for computing haptic forces to implement boundary constraints and a haptic control model (HMa) for computing haptic forces to implement active constraints. These haptic control models (HMa, HMb) can be separate or combined. When separate, as shown, one or more haptic object(s) (Hobj) is inputted into each haptic control model (HMa, HMb). The haptic object (Hobj) may be the same or different for each model. Either of these haptic control models can be implemented using any suitable control scheme, including but not limited to: a spring-damper model; a proportional-derivative (PD) model; or an impulse model. The active constraint haptic control model (HMa) generates haptic forces (F_{active}) that are intended to actively constrain specified DOF of the surgical tool, i.e., pursuant to the haptic object definition. The boundary constraint haptic control model (HMb) generates reactive haptic forces (F_{reactive}) that are configured to reduce interaction between the end effector 22 and the virtual boundary 71 or haptic object geometry if such interaction is present or imminent. Impulse modeling can be used to compute reactive haptic forces without requiring boundary penetration.

Numerous examples of active and boundary constraints are contemplated. For boundary constraints, the haptic objects (Hobj) can be implemented with a variety of shapes including some predefined geometric constraints such as a plane, a line, or a volume (e.g., cylinder, cone, or box) haptic. Haptic object shape can be also defined more generically using a polygon mesh constraint which is composed of a set of triangles that are connected by their common edges and vertices. For example, the haptic object (Hobj) for the boundary constraint can be implemented as a line haptic defined by a mesh volume.

For active constraints, the haptic objects (Hobj) can be implemented by DOF restrictions on the end effector 22, which can mimic an intended haptic geometry. In one example, the haptic object (HobJ) for the active constraint is a haptic plane and the haptic control model (HMa) is configured to compute haptic forces (F_{active}) that are intended to constrain at least three specified DOF of the end effector 22 relative to the haptic plane. In this case, the at least three specified DOF comprise two rotational DOF (Rx, Ry) and at least one translational DOF (Tz). This way, the end effector 22 is allowed to translate in and out of the plane (Tx), translate left or right in the plane (Ty), and rotate in within the plane (Rz), while respecting the 3DOF of the planar constraint (Tz, Rx, Ry). Such planar constraints may be suitable for constraining a saw blade during resection, for example. Notably, the planar constraint can be up to 6DOF. The planar constraint may require less DOF when the robotic system employs a passive joint or planarly extending joint.

The haptic object (HobJ) for the active constraint can also be a haptic line. The haptic control model (HMa) is configured to compute the haptic forces (F_{active}) that are intended to constrain at least four specified DOF of the end effector 22 relative to the haptic line. The at least four specified DOF can include at least two rotational DOF (Ry, Rz) and two translational DOF (Ty, Tz). This way, the end effector 22 is allowed to translate up/down the haptic line (Tx) and rotate (about the tool axis, Rx) corresponding to the haptic line, while respecting the 4DOF of the line constraint (Ty, Tz, Ry, Rz). Similarly, the line constraint can be more restrictive, if desired, e.g., up to 6DOF. In the case in which the active constraint is implemented by a haptic volume, the haptic control model (HMa) is configured to compute haptic forces (F_{active}) intended to constrain up to three specified DOF of the end effector 22 relative to the haptic volume. For example, up to three rotational DOF can constrained. Notably, the planar constraint can be up to 6DOF. Such line constraints may be suitable for constraining a cutting bur, router, drill, screwdriver, or any other tool with a straight shaft, for example.

Another haptic control model is a predefined arm feel model (HMf). The predefined arm feel model (HMf) is configured to compute haptic forces (F_{damp}) that are intended to define a baseline physical feel of the manipulator 14 to the human (patient/surgeon). The physical feel is how sluggish or responsive the manipulator 14 will feel to the human in response to the human applying forces to the manipulator 14/end effector 22. The predefined arm feel model (HMf) may utilize any suitable control scheme to compute haptic forces, including but not limited to: a spring-damper model; a proportional-derivative (PD) model; or an impulse model. The predefined arm feel may be defined by specifying a virtual mass of the virtual rigid body model of the end effector 22 and/or by specifying spring/damping parameters of the manipulator 14. The predefined arm feel may be set a factory default setting or a setting that is adjustable by a human operator during the medical/surgical procedure. The predefined arm feel may be activated for all collaborative interactions of the manipulator 14. The above-described active and boundary constraints may be added to the predefined "feel." For example, when the end effector 22 enters a zone, in which the active/boundary constraints are activated, the human will experience an arm feel that is combination of the predefined "feel" in addition to the active/boundary constraints (when utilized).

The haptic forces (F_{active}, F_{reactive}, F_{damp}) from the above-described haptic control models (HMa, HMb, HMf) can be combined to generate a total haptic force. If the described force/torque sensor (S) is utilized (as shown in FIG. 6), the force/torque values obtained from the sensor (S) can be combined with the total haptic force to generate a total force. Based on the total force, the control system 60 (or manipulator controller 26 and/or motion controller 76) is configured to generate commanded joint torques (τ) to control the respective joint(s) (J) of the manipulator 14. If the total force includes active haptic forces (F_{active}), the commanded joint torques will include components to move to constraint poses in attempt to actively constrain the specified DOF of the surgical tool. If the total force includes reactive haptic forces (F_{reactive}), the commanded joint torques will include components to alleviate tool-boundary interaction. If the total force includes forces from the force/torque sensor S, the commanded joint torques will include components to move the end effector 22 in a manner that mimics the surgeon's interaction with the end effector 22, while respecting the active and boundary constraints. This type of computation can be part of an admittance type system that optionally utilizes the behavior controller 74 to implement a virtual rigid body simulation of the total force to determine commanded positions or joint torques.

The control system 60 implements a control law or "behavior policy" that defines the physical feel of the manipulator 14 during collaborative interaction between the human and the manipulator 14. As shown in FIG. 6, the behavior policy is defined, at least in part, based on the haptic control models (active constraint model HMa, boundary constraint model HMb, and predefined arm feel model HMf) described above. Each of these haptic control models (HMa, HMb, HMf) can have an influence on the combined manipulator 14 feel. Some of the haptic control models, such as the active constraint model HMa and boundary constraint model HMb may not be active. In such cases, the behavior policy may not include the output of these models under such conditions. The behavior policy outputs the total haptic force, described above.

With continued reference to FIG. 6, the computed joint torque (τ) pursuant to the haptic control model calculations may optionally be combined with additional feed forward torques (τ_{ff}). Feed forward torques can include gravity torques and forces required for joints to maintain their positions in the specified gravity. Gravity torques and forces can be computed from the actual measured joint positions (qₘ). Feed forward torques can also include joint damping torques to smooth out movements and prevent oscillations of the joints. Damping torques can be calculated from the velocity (q dot m) of the joints. The feed forward torques (τ_{ff}), and the commanded joint torques (τ) from the haptic constraints can be combined into a total commanded torque (τₜ) by which the control system 60 will command the joints.

Forward kinematic calculations can be used to determine the measured pose (Xₘ) of the end effector 22 or TCP. The measured pose (Xₘ) of the end effector 22 can be fed back into the control loop to determine the difference between the measured pose (Xₘ) and the desired pose (X_{d}) of the surgical tool relative to the haptic objects (Hobj). The difference of this comparison (ΔX) is used in the next time step to re-compute the necessary boundary and active constraints, and this process can repeat for multiple time steps. The processes described herein can be iteratively performed and repeated for any number of time steps during run-time of the manipulator 14 and can do so depending on presence or absence of conditions, such as detected human interaction, surgical steps, operation of certain modes of operation (e.g., manual, automated, semi-autonomous, guided-manual), etc.

### II. Techniques for Employing Machine Learning Models to Determine User Interaction Style

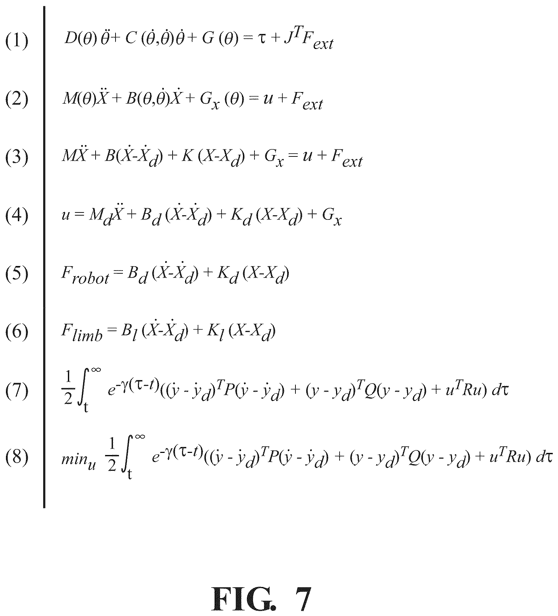

With reference to FIGS. 7-13, described herein are systems, methods, and non-transitory computer readable media (computer program products) for employing machine learning models to determine (predict/estimate/classify/characterize) an interaction style of the human user in collaborating with the manipulator 14. As described above, the techniques can apply to a collaborative surgical robot, wherein the human user is a surgeon or a surgical technician. In other examples, the techniques can apply to a collaborative rehabilitation robot, or any other type of medical robot, wherein the human user is a patient.

### A. Example Problem Statement

In this sub-section, we describe an example technical problem addressed by the technical solutions described herein. The details provided below are one way of characterizing the technical problem and are not intended to limit the scope of the design. Indeed, there are numerous other technical problems being solved, such as those described throughout this document.

We begin by expressing the dynamic model of the manipulator 14 as set forth in equation (1) in FIG. 7. We can rewrite the dynamics equation in Cartesian space, as set forth in equation (2) in FIG. 7, where M and B represent the Mass matrix and damper matrix of the manipulator 14 which are dependent on the robot arm configuration (joint positions and velocities). The dynamics of the manipulator 14 can be generalized to be a mass-spring-damper model where there exists a desired position (and/or desired velocity) to follow, as set forth in equation (3). In robotics-assisted surgical/medical applications, the behavior policy for the collaborative robot arm (u) is designed such that all the gravity impact of the heavy arm is compensated (e.g., by the feed forward torque). In addition to that, the manipulator 14 is felt to have a pre-defined (desired) mass and damper feeling (Md and Bd), as defined by the predefined arm feel control model (HMf). Moreover, the feel of the manipulator 14, in contact with a virtual object (as defined by the boundary constraint control model (HMb) or when maintaining an active constraint (such as where a desired trajectory is defined) is defined by the active constraint control model (HMa) and will be set to a pre-defined value of Kd. In view of the foregoing, the behavior policy can be generally defined as set forth in equation (4).

With this pre-defined behavior policy, the gravity impact of the manipulator 14 is compensated while the manipulator 14 is felt like a mass-spring-damper with some pre-defined values. In most cases, the acceleration terms can be ignored as surgical/medical robots typically operate at low speeds with carefully controlled motions, minimizing acceleration effects. Moreover, the gravity compensation is usually applied as the minimum feed-forward torque for these platforms. Therefore, we can focus on the behavior policy as a spring-damper model where the spring and damping constants can be assigned differently under different policies. The way the manipulator 14 is to be physically felt by the user during human-robot interaction can be expressed as set forth in equation (5).

On the other hand, the force applied by the human limb during human-robot interaction can be modeled as set forth in equation (6) (ignoring the acceleration-dependent term). Now, if the pre-defined arm feel (*B_{d} , K_{d}*) matches the limb impedance model (*Bₗ , Kₗ*), the optimal interaction behavior occurs. However, if there exist a discrepancy between the two, then the collaborative robot is not optimally designed for that particular human. In equations (5) and (6), the first term of the equations relates to how the robot/limb responds to the motion when a desired velocity is set. As mentioned earlier, when the pre-defined robot damping (the resistance against the motion) matches the damping of the limb dynamics, the optimal motion occurs. However, if the human user finds it difficult to move the arm (sluggish feeling) it means the pre-defined damping on the robot arm is set larger than that individual's limb damping. On the other hand, if the human uses the robot arm aggressively it means the robot arm may not have enough damping for that particular human and the robot may be felt with the lack of enough control during the collaborative interaction.

The second term of the equations (5) and (6) represents how the robot/limb responds to virtual fixtures and/or active constrains where a desired motion is set. This is a spring model demonstrating how strong either side feels (analogous to an arm-wrestling contest). In the context of robotic assistance, when there is a tie (*K_{d} = Kₗ*), this means that the virtual constraints are perfectly designed for that individual within his strength level. When *K_{d} < Kₗ* it means the individual is stronger (heavy-handed) and the chance of fighting against the applied haptic is high.

In the surgical context, haptic boundaries are designed to help surgeons to only resect the area of interest inside the boundaries. When the surgeon is heavy-handed the chance of over-resecting would be high resulting in inaccurate resections/cuts. With active constraints, if the surgeon keeps fighting against the constraint the chance of getting the robot arm unstable would be also increased which is a safety concern for both the patient and surgeon.

### B. Introduction

The techniques herein involve the control system 60 acquiring robotic data, e.g., during collaborative interaction between the human and the manipulator 14. The control system 60 implements a machine learning model (MLM) that is configured to receive and process the robotic data to determine an interaction style of the human in collaborating with the manipulator 14. In some cases, the control system 60 initially controls the manipulator according to a (predefined) behavior policy defining the physical feel of the manipulator 14. As described above, this behavior policy can be based on any one or more of the haptic control models (HMa, HMb, HMf). Then, the control system 60 can update the behavior policy based on the interaction style to customize the physical feel of the manipulator 14 to the interaction style. In other cases, the control system 60 defines the behavior policy based on the interaction style (assuming there was no initial behavior policy). As such, the control system 60 can update/refine the existing behavior policy, or define the behavior policy, based on the interaction style.

As will be understood from the disclosure, the described techniques provide numerous technical and practical solutions. For example, by determining the user interaction style with the machine learning model (MLM), the physical feel of the manipulator 14 is customized to the human user thereby ensuring that the feel is specifically suited for the particular human user. The interaction style can be determined and updated in real-time, to account for changing conditions and human interaction, without relying on a "one size fits all" or non-adaptable arm feel model. The interaction style can take into account any one or more of the following: human muscle strength, human applied force, human resistance, light/heavy-handedness, stiffness/damping of a human limb, burden on the human, human fatigue, human effort, human power, workload/work of the human, or the like. Moreover, although these value-related factors may be considered, the interaction style determination can be a higher-order prediction/estimation of the human's behavior. The machine learning model can characterize virtually any behavior to describe the user's interaction. Inferring human behavior provides a more intelligent and dynamic (non-linear) estimation of the human interaction style. To elaborate, the interaction style can account for whether the human behavior is patient/impatient, decisive/hesitant, aggressive/relaxed, fast-acting/slow-acting, stable/unstable, or the like. For example, the human user may be physically strong, yet quite cautious in their collaborative interaction. On the other hand, the human user may be physically weak, yet quite aggressive in their collaborative interaction. In some cases, the human user can consciously or unconsciously change their interaction style during the collaborative interaction. For example, the human's aggressiveness or strength may progressively decrease during the collaborative interaction due to fatigue or exhaustion. Prior techniques of measuring force or even reacting to physical strength would not truly account for these various behaviors of the human user. On other hand, the described solutions are adapted to dynamically optimize the "feel" of the robot based on the true nature of the human's interaction style, as well as the context in which the human is operating or collaborating with the robot. With the power of AI, this "on-the-fly" approach can also monitor any deterioration in human strength, due to fatigue, lengthy procedure, etc., and make the appropriate adjustments to the robot feel setting to adaptively provide a better user experience during collaborative interaction. In turn, the techniques provide technical solutions to improve not only the human user experience, but also the robotic performance.

In surgical settings, the manipulator 14 will exhibit more predictable response and greater accuracy by accounting for the user's interaction style. Increased accuracy can result in better surgical outcomes for the patient. The solutions herein can also avoid compromising virtual haptic tuning as the conventional haptic tuning prioritizes robotic arm stability over accuracy. By adaptively tuning the behavior policy according to surgeon interaction style, the techniques can increase the stiffness of the virtual haptics for certain styles to improve the accuracy while reducing the risk of vibration. The described techniques can help achieve an optimal surgeon-arm interaction based on their comfort zone. This includes an individualized feel of the robot arm when the surgeon manipulates the robotic arm in free zone as well as an adaptive feel within their comfort zone for a specific application and specific cutting tool during bone resection. In the medical setting, the manipulator 14 can exhibit a customized feel for the patient to provide full therapeutic benefits and faster recovery time, while avoiding potentially reaggravated patient injury.

Moreover, as will be understood below, contextual information about the medical/surgical procedure/environment/tools can be considered such that the determined interaction style can be specifically tailored to the given context. In turn, the robot will behave in a manner that is convenient/ergonomic/comfortable to the human user, regardless of the situation or conditions present. For example, a surgeon can have a first interaction style wherein the surgeon is careful/slow acting during a reaming step for THA, and the surgeon can have a second interaction style wherein the surgeon is aggressive/fast-acting during an impaction step for THA. The techniques can update the behavior policy at different times to account for both types of interactions. In some cases, the user interaction style can favor obtaining a more accurate robotic operation over optimizing the feel of the user. For example, if the surgeon style is aggressive, the robotic arm feel can be adjusted to add more damping and create more resistance against the aggressive motion. In another example, if the surgeon interacts with a forceful style, the robot stiffness can be reduced to avoid unexpected robot instability and promote safer robotic operation. Other benefits and advantages will be understood from the description below.

### C. Machine Learning Model

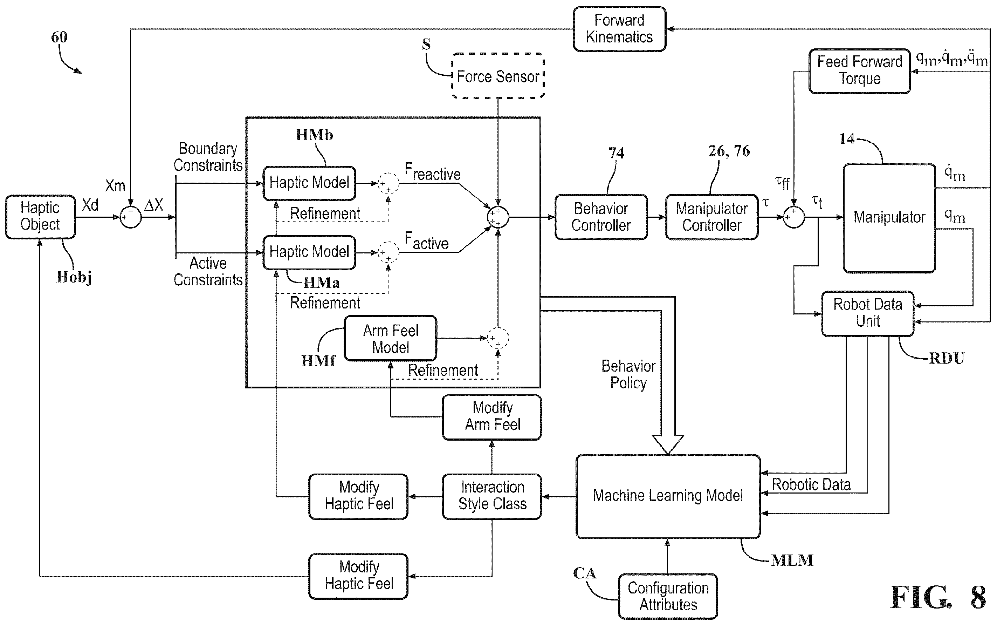

With reference to FIG. 8, provided is an example block diagram of control processes that can be performed by the described techniques for determining the user interaction style. Figure 8 provides the control scheme of FIG. 6 modified by inclusion of the machine learning model (MLM) and other processes to enable the determination of the user interaction style.

Throughout this description, the steps or processes of the control schemes will be described as being performed by the control system 60. As described, the control system 60 can include any one or more of the described controllers or components of the system 10. One or more machine learning models (MLM) can be added or incorporated to the control system 60 to refine aspects of the control system 60. In other implementations, the machine learning model(s) (MLM) can used as a substitute for components/software of the control system 60. For example, the machine learning model(s) (MLM) can substitute for the haptic control models (HMa, HMb), behavior controller 74, and/or the motion controller 76.

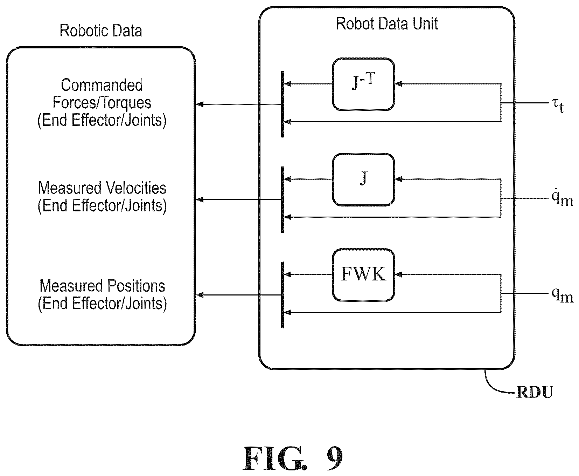

As shown in FIG. 8, the machine learning model (MLM) can receive the behavior policy from one or more of the haptic control models (HMa, HMb, HMf). The machine learning model (MLM) also receives robotic data from the manipulator 14. In some cases, the robotic data can be processed by a robot data unit (RDU), also shown in FIG. 9. Configuration attributes (CA) can be inputted into the machine learning model (MLM) to provide context about the interaction. Based on this inputted data, the machine learning model (MLM) determines the user interaction style. The interaction style is then used to update the behavior policy. To do so, the control system 60 can update/refine the arm feel control model (HMf), the active constraint control model (HMa), and/or the boundary constraint control model (HMb). The control system 60 can also update the haptic object (Hobj) which feeds into the active/boundary constraint models (HMa, HMb).

In cases where there is no initial behavior policy, the machine learning model (MLM) can process the robotic data to determine the interaction style. Then, the control system 60 can define the behavior policy based on the interaction style or define haptic forces based on the interaction style. In an alternative implementation, the control system 60 can refine the haptic forces of any one or more of the haptic control models (HMa, HMb, HMf) based on the interaction style. In other words, instead of changing the control law of one or more of the haptic models (HMa, HMb, HMf), the control system 60 can add a refinement force to the respective model(s).

### 1. Robotic Data

As described above, the machine learning model (MLM) receives and processes robotic data from the manipulator 14. The robotic data inputted into the machine learning model (MLM) can be several types of data to provide information about the collaborative interaction between the human and the manipulator 14. The robotic data can be acquired during the collaborative interaction. In some cases, robotic data can also be acquired from prior collaborative interactions (whether it be interactions from the particular human or from others).

Turning to FIG 8, the robot data unit (RDU) is illustrated to process the various types of robotic data. The robot data unit (RDU) is part of the control system 60 and can be a separate controller or part of any of the described controllers, such as the manipulator controller 26, motion controller 76, behavior controller 74, or the like. The robot data unit (RDU) is configured to provide the machine learning model (MLM) with commanded forces/torques, measured velocities, and measured positions/poses.

The commanded forces/torques includes forces/torques that are commanded by the control system 60 to the control the manipulator 14, e.g., during the collaborative interaction. Commanded forces are forces commanded to move the end effector 22 in task space. Commanded forces are forces commanded to move the joints (J) of the manipulator 14 in joint space. The commanded forces/torques can be obtained by the total commanded torque (τₜ), which can include a combination of the feed forward torques (τ_{ff}), and the commanded joint torques (τ) from the one or more haptic constraint models (HMa, HMb, HMf). To obtain the commanded force, the robot data unit (RDU) can apply an inverse Jacobian transposed transformation to the total commanded torque (τₜ). In some cases, the commanded forces/torques may not be based on the total commanded torque (τₜ), but based on components of the total, such as based on only the commanded joint torques (τ) from the one or more haptic constraint models (HMa, HMb, HMf) (without feed forward torques). Any number of N previous samples of commanded forces/torques can be acquired.

The measured velocities are actual or predicted velocities of the manipulator 14 that have been measured, e.g., during the collaborative interaction. The measured velocities can be velocities of the end effector 22 or velocities of any one or more of the joints (J) of the manipulator 14. Such velocities may be measured at, or otherwise derived from movement of, the end effector/joints. Measured joint velocities (q̇m) can be obtained from the sensors on the robotic arm, such as joint encoders, or the like. The measured end effector velocities can be determined based on the measured joint velocities (qm) of the manipulator 14, as well. The robot data unit (RDU) can apply a Jacobian transform to mathematically relate the measured joint velocities (qm) to the measured velocity of the end effector 22. End effector velocities can be measured using other methods, such as from tracking data obtained the navigation system 32, inertial sensors provided on the end effector 22, or the like. In other examples, the control system 60 can obtain the measured velocities by computing the derivative of the measured displacements of the joints or end effector. Any number of N previous samples of measured velocities can be acquired. As it relates to these values, the term "measured" is synonymous with "prior" and the following description may use these terms interchangeably.

The measured poses/positions are prior poses/positions of components of the manipulator 14. In one implementation, the measured poses/positions are related to the task space of the manipulator 14. Here, the measured poses/positions can include previously measured poses (Xm) of the end effector 22. The measured end effector pose(s) can be derived by obtaining prior measured joint positions/angles (qm) of the joints (J) of the manipulator 14 and applying the measured joint positions/angles to a forward kinematics model of the manipulator 14. Other sensing means, such as tracking data of the tool 20 derived from the navigation system 32 or inertial or motion sensors on the surgical tool 20 can be utilized to determine the measured poses (Xm). Additionally, or alternatively, the measured poses/positions can be related to the joint space of the manipulator 14. For example, measured positions can include prior joint positions/angles (qm) that were measured at the joint(s) of the manipulator 14 after application of the computed joint torques (τ, τt) for the respective joint(s). The control system 60 controls the joints of the manipulator 14 in accordance with the computed joint torques to move the joints (J) to their respective joint positions/angles (qm). The measured joint positions/angles (qm) can be measured using any suitable means, such as joint encoders, kinematic analysis, potentiometers, inertial sensors, navigation system-based tracking, machine vision tracking, or the like. Any number of N previous samples of the measured pose/positions can be acquired.

Another type of robotic data that can be inputted into the machine learning model (MLM) is target, reference, or desired value(s) (Xd, qd). The desired value(s) (Xd, qd) can be predetermined, generated, inferred, or predicted by the control system 60, robot data unit (RDU), or machine learning model (MLM). The desired value(s) (Xd, qd) can represent a desired behavior of the manipulator 14 and/or end effector 22. In one example, the desired value can be a desired pose or position of components of the manipulator 14 and/or end effector 22. For instance, the desired value can be one or more desired pose(s) (Xd) for the end effector 22. In another example, the desired value can be desired joint positions (qd) for one or more joint(s) of the manipulator 14. Depending on the type of end effector 22 or surgical/medical procedure, for example, the desired values (Xd, qd) can be defined for any suitable purpose.

The machine learning model (MLM) is also configured to receive robotic data that represents future or expected values. Such values can be those that predict or represent any future/expected behavior of any one or more components of the manipulator 14 and/or end effector 22. The future/expected behavior may be ideal or may be sub-optimal (trial and error). In one example, the future/expected values can be future poses/positions of components of the manipulator 14. For instance, the future/expected values can be one or more future pose(s) for the end effector 22. In another example, the future/expected values can be future joint positions for one or more joint(s) of the manipulator 14. The future/expected values can be predetermined, generated, inferred, or predicted by the control system 60 or machine learning model (MLM). In some cases, future/expected values can be derived from any of the prior (measured) position/pose values (Xm, qm). For example, the control system 60 and/or machine learning model (MLM) can employ any suitable algorithm or modeling to determine the future/expected values. Such algorithms or modeling may include regression models, neural networks, clustering models, or the like. Depending on the type of end effector 22 or surgical/medical procedure, for example, the future/expected values can be defined for any other suitable purpose.

### 2. Machine Learning Model Configurations

Having introduced various inputs into the machine learning model (MLM), we now introduce components/modules/features of the machine learning model (MLM) that are configured to process the inputs. The features of the machine learning model (MLM) can be implemented by any one or more components (e.g., controllers, processors, memory) of the control system 60. In some cases, it is contemplated that the machine learning model (MLM) can partially or fully be implemented in a remote controller or remote server that is remotely coupled to the manipulator 14. The remote controller/server can form part of the control system 60. For example, the described inputs can be transmitted to over the internet to the server for receipt by the machine learning model (MLM). The machine learning model (MLM) can remotely transmit control signals, behavior policy updates, or haptic force refinements over the internet to the manipulator 14 or control system 60.

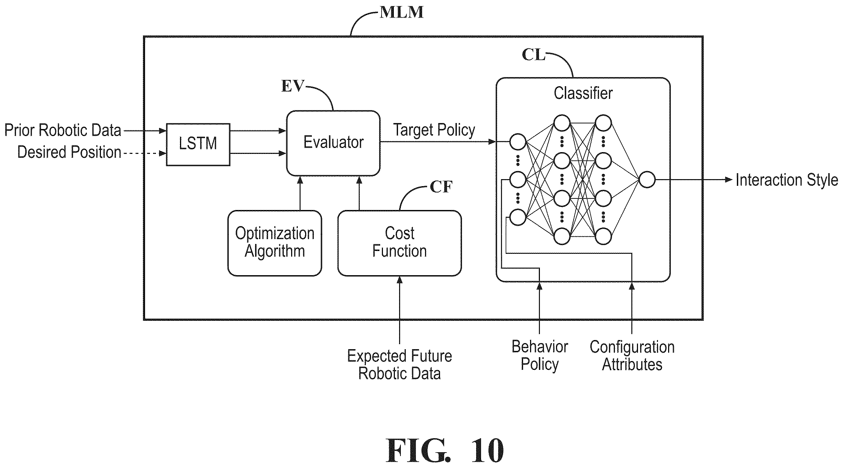

With reference to FIGS. 10, 12 and 13, the control system 60 and/or machine learning model (MLM) are configured to implement, process, and/or utilize an evaluator (EV), a classifier (CL), a cost function (CF), an optimization algorithm, and optionally, a long-short term memory LSTM. Each of these features will be described below.

Notably, using the machine learning model(s) MLM, the techniques described herein can provide "data-driven" solutions to robotic control. By data-driven, it is understood that the model for refining control of the manipulator 14 can be derived from real-world experiences of the manipulator 14 and/or end effector 22, without necessarily requiring a complete pre-programmed control model. The data-driven techniques can receive substantial amounts of prior robotic data to dynamically derive/update the behavior policy and evolve the control model over time to effectively learn optimal behavior for the manipulator 14 and/or end effector 22 given certain conditions. In some cases, the data-driven techniques can be individually tuned/trained or learn optimal behavior for specific robotic behaviors or surgical tasks, such as, but not limited to drilling a hole, cutting a plane, or adapting to the specific behaviors of the surgeon. Medical tasks can also be used for training, such as physical rehabilitation exercises, guided movements, and the like. The data-driven control model can adapt to compensate for unexpected situations and non-linearities. Data-driven techniques described herein can include but are not limited to model-free adaptive control (MFAC), model-free predictive control (MFPC), deep learning, reinforcement learning, deep reinforcement learning, integral reinforcement learning, imitation learning, or the like. It is also contemplated to utilize a novel approach to pure data-driven control using a variation of MFAC or MFPC that employs the described neural network(s). Although data-driven models are contemplated, it should be noted that the described techniques may be used with supervised learning, unsupervised learning, or with pre-programmed models and the solutions are not necessarily limited to data-driven control.

### a. Evaluator

The machine learning model (MLM) implements the evaluator (EV) to receive and process the input values, i.e., robotic data and desired pose/position data. In one example, the input values are directly inputted into the evaluator (EV). In another implementation, the input values are optionally pre-processed by a long short-term memory (LSTM) prior to being inputted into the evaluator (EV). The LSTM can be part of, separate from, or coupled to an input of, the machine learning model (MLM). The LSTM provides a dynamic memory cell for the vast amount of incoming measurement data. Using the LSTM, the machine learning model (MLM) can be injected with a mini-batch of historical time-delay data, including prior robotic data. The LSTM can include a recurrent neural network (RNN) that processes the prior robotic data values and modifies weights and biases of the RNN to learn long-term dependencies among the values and to selectively retain or discard the robotic data values. The RNN can be trained in a supervised or unsupervised fashion using sample robotic data measurements and by utilizing any suitable optimization algorithm, such as gradient descent, backpropagation through time (BPTT), or the like. In some cases, a recurrent neural network (RNN) can be used instead of an LSTM.

One objective of the evaluator (EV) is to explore or define a target policy based on the inputted data. The target policy can be optimally designed for a particular environment to result in a smooth interaction while injecting the minimum energy to the system. The target policy may be a target version of the behavior policy, which as described above, defines the physical feel of the manipulator 14. The target policies can be used as a strategy to control or refine actions of the manipulator 14. The target policy can define the decision-making strategy, rules or heuristics that are utilized to select actions for the controlling components of the manipulator 14. In one example, the target policy is an update control policy, which defines the rules used to update the various haptic control models. In another example, the target policy is specifically a refinement control policy, which defines the rules used to refine the haptic forces for the various haptic control models. The evaluator (EV) can determine the most optimal control policy to achieve a desired outcome. For the refinement control policy, the desired outcome may be, for example, to achieve the desired pose of the end effector 22 or the desired positions of the joints (J). When the machine learning model (MLM) is used to directly control the manipulator 14 (rather than to refine force/torque values), the target policy may be defined to obtain any other desired outcome such as, for example, to achieve the desired behavior of the end effector 22 and/or the desired behavior of the joints (J). Through the described process of evaluating prior values, the target policy can be iteratively updated as the machine learning model (MLM) continues learning from environmental conditions.

The evaluator (EV) can explore or define target control policies that optimize a cost function (CF). The cost function (CF) is a mathematical formula that is defined as a metric for the evaluator (EV) to derive the target policy such that it minimizes the cost function (CF). The expected/future robotic data value(s) can be inputted into the cost function (CF). Optionally, desired robotic data values can also be inputted into the cost function. The cost function (CF) can be also defined to evaluate the machine learning model (MLM) identification/prediction and to derive a policy update for the machine learning model (MLM) for increasing the accuracy of the future predictions. Using the cost function (CF), the machine learning model (MLM) can minimize any suitable variable, such as, but not limited to any one or more of the following: future residual errors, oscillation in end effector poses, and/or energy injected into the manipulator 14. The target policy can be iteratively adapted or optimized by the model error outputted by the cost function (CF).

In a data-driven control scheme, the prior robotic data can be collected to make an inference about the expected future data of a given system. This can be done as an extrapolation of the prior robotic data, or an advanced machine learning technique can be utilized to acquire a more accurate prediction of the expected future data. When the expected future data are available then the evaluator can form the cost function (CF) as set forth in equation (7) of FIG. 7. An optimization algorithm (as set forth in equation (8)) can be implemented to find a target policy that minimizes the cost function (CF). The first term in the cost function is *e*^{-*γ*(*τ-t*)} determining the long-term dependencies of the future robotic data and *y_{d}* and *ẏ_{d}* are the desired position and velocity data, respectively. Depending on how the collaborative robot is used, we may utilize one or both desired data terms in the cost function (CF). In the case of unconstrained manipulation of the robot arm, we only have a desired velocity of *ẏ_{d}* and the second term can be omitted from the cost function (CF) and the term of (*ẏ - ẏ_{d}*)*^{T}P*(*ẏ - ẏ_{d}*) indicates how aggressively the human moves the robot arm in its workspace. In the case of virtual haptics and active constraints, we mainly have the desired position of *y_{d}* while the desired velocity of *ẏ_{d}* is set to zero. Therefore, the term of (*y - Y_{d}*)*^{T}Q*(*y - y_{d}*) is as an indication of how forceful the human is while interacting with the virtual haptics and active constraints (a measure of human's strength). The optimization problem in equation (8) is to find the policy (u) that minimizes any deviation from the desired position *y_{d}* and/or from the desired velocity *ẏ_{d}.*

As will be described in the examples below, the evaluator (EV) can take various configurations or forms depending on the nature of the machine learning model (MLM). The evaluator (EV) can be included in various architectures, such as, but not limited to model-free adaptive control (MFAC), model predictive control (MPC), reinforcement learning (RL), or deep reinforcement learning (DRL). In MFAC, the machine learning model (MLM) can estimate the target control policy. In MPC, the machine learning model (MLM) can predict the target control policy. In reinforcement learning, the machine learning model (MLM) can evaluate the target control policy.

In one example, the evaluator (EV) can include a neural network (NNev) that is responsible, in part, or in whole, for defining the target control policy. In one implementation, the neural network (NNev) can receive, at the input layer, the prior commanded force/torque value(s) and the prior measured pose/position value(s), or variations thereof. In other instances, the neural network (NNev) can additionally receive, at the input layer, prior measured velocities values (of the end effector and/or joints), as well as optionally, the desired position/pose values (of the end effector and/or joints). Again, the LSTM/RNN can be used as a gateway/filter for the robotic data provided to the neural network (NNev) of the evaluator (EV). The neural network (NNev) can include any suitable number of hidden layers and can be any suitable type of neural network architecture, including but not limited to: deep-learning neural network, a deep-reinforcement learning neural network, a recurrent neural network (RNN), a multilayer perceptron (MLP), a feed-forward neural network, a convolutional neural network (CNN), or the like. The output layer of the neural network (NNev) can produce various results depending on the network configuration. In one example, the output layer can produce the target control policy or an update law that is used to evaluate/modify the behavior policy. In another example, the output layer produces an identification of a state of the manipulator 14 or a prediction/estimate of the state of the manipulator 14. The identified or predicted state values can be subjected to optimization using the cost function (CF) and/or other optimization constraints to define the target control policy.

### b. Classifier

As shown in FIGS. 10, 12 and 13, the classifier (CL) is a part of the machine learning model (MLM) that utilizes the target control policy generated by the evaluator (EV). The classifier (CL) may be incorporated into the evaluator (EV) or may be implemented separately therefrom. The classifier (CL) also receives as an input the behavior policy and configuration attributes (which will be described below). The classifier (CL) is configured to compare the behavior policy and the target policy. Based on the comparison, the classifier (CL) determines the interaction style of the human in collaborating with the manipulator.

As will be described in the examples below, the classifier (CL) can take various configurations or forms depending on the nature of the machine learning model (MLM). The classifier (CL) can be included in various architectures, such as, but not limited to model-free adaptive control (MFAC), model predictive control (MPC), and reinforcement learning (RL).

The classifier (CL) can include a neural network (NNcl) that is responsible, in part, or in whole, for determining the updated behavior policy to influence the physical feel of manipulator 14. The neural network (NNcl) can include any suitable number of hidden layers and can be any suitable type of neural network architecture, including but not limited to: deep-learning neural network, a deep-reinforcement learning neural network, a recurrent neural network (RNN), a multilayer perceptron (MLP), a feed-forward neural network, a convolutional neural network (CNN), or the like.

### i. Configuration Attributes

As shown in FIGS. 8, 10, 12 and 13, machine learning model (MLM) can utilize configuration attributes (CA) to better understand a context of the collaborative interaction between the human and the manipulator 14. Using the configuration attributes, the classifier (CL) can facilitate determination of the interaction style of the human specifically in view of the context defined by the one or more configuration attributes. The context provided by the configuration attributes can provide the machine learning model (MLM) with additional insight about how the human may be interacting with the manipulator 14. This context can provide a higher-order, customized, understanding of the collaborative interaction between the human and the manipulator 14.

The one or more configuration attributes specify: environmental attributes, robotic control attributes, application attributes, and/or robotic tool attributes. The environmental attributes can identify features of the environment in which the human is interacting with the end effector 22. The robotic control attributes specify attributes, parameters, operation, and/or control, modes of the manipulator 14. The application attributes specify attributes/parameters of surgical/medical application performed on/with a patient. The tool attributes specify features, parameters, operation, control, and/or the type of end effector 22 attached to the manipulator 14.

The control system 60 is configured to obtain the configuration attributes from any suitable source and at any suitable time. For example, the control system 60 can obtain configuration attributes from any of the described components/controllers, such as the navigation system 32, clinical application 80, manipulator controller 26, behavior controller 74, or the like. The configuration attributes can also be sent to the control system 60 from a remote sensor. Configuration attributes can be factory or default settings that can be acquired by the control system 60. The configuration attributes are subject to change during the collaborative interaction. For example, a new surgical step may require a different type of surgical tool, robotic control type, and/or type of application (e.g., screwing instead of drilling). As such, the control system 60 can receive updates/changes to any of the configuration attributes over time.

In the surgical context, the configuration attributes can specify surgical environment attributes, a surgical robot control attributes, surgical application attributes, and/or surgical tool attribute. Surgical environment attributes can specify the surgical environment of the end effector 22 in the surgical procedure. For example, surgical environment attributes can specify whether the end effector 22 is in free zone, haptic zone, resection zone, allowed region, forbidden region, or within/outside a virtual boundary. The surgical environment attributes may also include the level of task difficulty/workload. Example methods of predicting task difficulty or workload can be like that described in US Patent App. No. 19/339,378, filed September 25, 2025, entitled "Techniques For Estimating Deflection Of A Surgical Robotic Arm", the entire contents of which are hereby incorporated by reference. Surgical environment attributes can also specify with what type of object the end effector 22 is interacting. For example, the end effector 22 may interact with soft tissue, bone, or the like. Example methods of predicting tool interactions can be like that described in US Patent App. No. 19/339,389, filed September 25, 2025, entitled "Robotic Surgical Systems And Methods Employing Machine Learning Models To Characterize Tool Interactions", the entire contents of which are hereby incorporated by reference. Other details that can be specified by the surgical environment attributes can include surgical planning details, implant information, incision size, retractor locations, and locations of various objects in the operating room (which can be determined by the navigation system), or the like.

The surgical robot control parameters can specify the mode of operation of the manipulator 14 (e.g., autonomous, semi-autonomous, manual, guided manual, free mode, etc.). The surgical robot control attributes can also specify whether the active/boundary constraints are active/inactive and features about these constraints (e.g., what degrees of freedom are actively constrained, or where boundaries are located, etc.). Other details that can be specified by the surgical robot control attributes can include preferred/predetermined poses of the robotic arm, tool path TP details, force sensor (S) sensitivity data, whether the manipulator 14 is an admittance control system or impedance control system, or the like.

The surgical application parameters can specify details of the surgical procedure or sub-steps of the procedure. The surgical application attributes can specify whether the end effector 22 is being used for performing a planar cut, screw placement, impaction, reaming, drilling, routing, milling, sculpting, or any other action that involves manipulation of a surgical site. In some instances, the surgical application attributes specify that the end effector 22 is being used to perform a robot registration procedure, or that the end effector 22 is being aligned to a desired position. The surgical application context can provide important insight for the interaction style determination as surgeons will collaborative differently depending on the given procedure. Other details that can be specified by the surgical application context can include whether procedure is navigated, an average duration of a surgical step, and the like.

The surgical tool parameters can specify features/parameters of the surgical end effector 22 attached to the manipulator 14. For example, the type of the end effector 22 can be specified, such as a rotary cutting burr, drill, saw, router, impactor, reamer, tool guide, pin-driver, or the like. The type of tool can impact the nature and duration of the collaborative interaction. For instance, the collaborative interaction of the surgeon in performing a planar cut will be different depending on whether the planar cut is being performed with a saw or a rotary cutting burr.

In the medical context, the configuration attributes can specify rehabilitation robot control attributes, a rehabilitation application attributes, and/or rehabilitation end effector attributes. The rehabilitation robot control attributes can specify any of the described robot control attributes, as well as whether the rehabilitation robot is an assistive system (to aid the patient in performing a movement) or a resistive system (to resist the patient to help the patient develop functional strength). The rehabilitation application attributes can specify the attributes of body part(s) being rehabilitated. For example, the rehabilitation application attributes can specify whether the body part(s) is/are: a limb, a leg or legs, an arm or arms, an upper body, a lower body, and what side (left/right) the body part is located. The rehabilitation application attributes can also specify the known information about the patient's body, such as range of motion, functional strength, pre-existing conditions, known locations of pain, impingement information, anatomical joint parameters, or the like. This information can be obtained from prior therapies or evaluations of the patient. Before or during collaboration with the rehabilitation robot, wearable sensors can be attached to the patient to relevant information. The rehabilitation end effector attributes can specify the details of the end effector 22 attached to the manipulator 14. For example, the end effector 22 can be hand-held, limb attached, a limb holder, an upper body holder, a lower body holder, the nature in which the limb is attached, or the like.

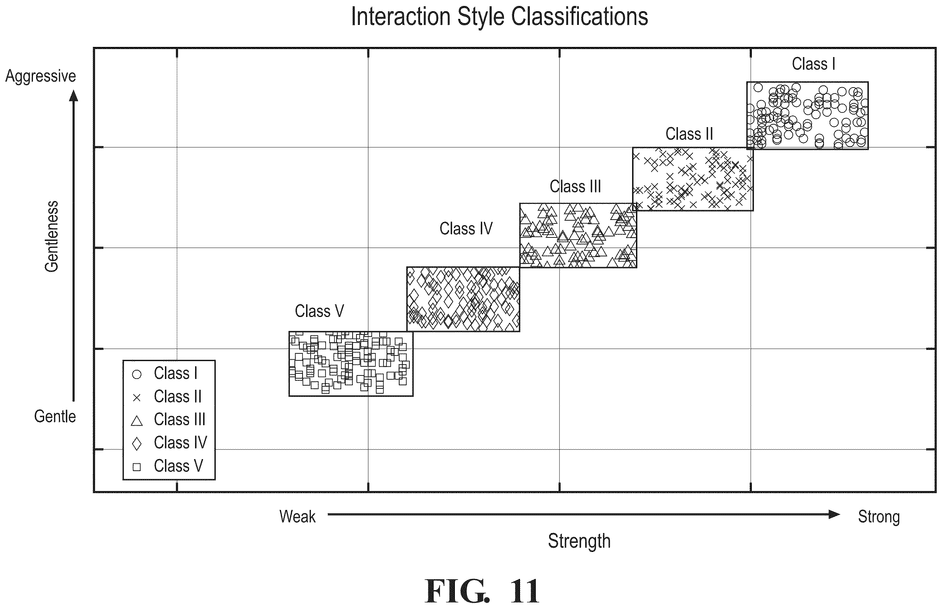

FIG. 11 is a chart demonstrating an example of how the machine learning model (MLM) can classify the interaction style of the human user. In this example, the machine learning model (MLM) processes the robotic data to make predictions about the gentleness of the human compared with the strength of the human. In this example, the machine learning model (MLM) can classify the interaction style into five different classes. The Class I interaction style accounts for situations where the human user is very strong and exhibits overly aggressive interaction. On the other hand, the Class V interaction style accounts for situations where the human user is very weak and exhibits very gentle interaction. Although five classes are shown in the chart, any number of classifications can be made, and such classifications need not be linearly arranged. Moreover, although strength and aggressiveness are considered in the chart, numerous other factors/values/behaviors can additionally or alternatively be considered when making such classifications. Additionally, the configuration attributes can inject context to enable the machine learning model (MLM) to determine a massive number of classifications, e.g., 500 or more interaction styles. For example, there can be subclass of interaction styles for free-zone interaction, saw-cutting interactions, burr cutting interactions, hard-bone interactions, reaming interactions, impaction interactions, etc. Countless possibilities are envisioned.

Numerous interaction style determinations can be made during collaborative interaction of the user, and these interaction style determinations can change over time. As the machine learning model (MLM) continues to learn from the robotic data and collaborative interactions, the machine learning model (MLM) will be able to make interaction style determinations with greater and greater accuracy during any procedure, or for any subsequent deployment of the machine learning model (MLM).

Once the machine learning model (MLM) determines the interaction style, the control system 60 is configured to define/refine (update) the behavior policy (as shown in FIG. 8). As described above, the behavior policy determines the physical feel of the manipulator 14 through one or more haptic models, including an active constraint haptic model (HMa), a boundary constraint haptic model (HMb), and a predetermined arm feel model (HMf), each of which are configured to produce the respective haptic forces (F_{active}, F_{reactive}, F_{damp}). By defining/refining the behavior policy, the machine learning model (MLM) can effectively customize the physical feel of the manipulator to the determined interaction style. The control system 60 can refine the behavior policy by updating any one or more of the haptic control models (HMa, HMb, HMf) and/or by refining any one or more of the respective haptic forces (F_{active}, F_{reactive}, F_{damp}). Such refinement is appropriate where the behavior policy exists before determination of the user interaction style. On the other hand, if no behavior policy exists before determination of the user interaction style, the control system 60 can define the behavior policy. The control system 60 can define the behavior policy by defining any one or more of the haptic control models (HMa, HMb, HMf) and/or by defining any one or more of the respective haptic forces (F_{active}, F_{reactive}, F_{damp}). The control system 60 can also define/refine the haptic object (Hobj) which feeds into the active/boundary constraint models (HMa, HMb).

The control system 60 can perform various actions to refine the active constraint control model (HMa) based on the interaction style. In one example, the control system 60 adjusts constraint parameters of any of the specified degrees of freedom which are being/to be constrained by the active constraints. The constraint parameters can be stiffness/damping parameters of any of the specified degrees of freedom of movement. In another example, constraint parameters can be impulse parameters of any of the specified degrees of freedom of movement. For example, if the surgeon is heavy-handed in the +X direction, the control system 60 can alleviate active constraints in this direction. In some cases, the control system 60 can refine the active constraint control model (HMa) by re-specifying the degrees of freedom of movement that are to be constrained for the end effector 22. For example, instead of constraints applied to the two rotational DOF and one translational DOF, the constraints can be re-specified to one rotational DOF and two translational DOF. Moreover, as described, the haptic object (Hobj) can be utilized in computation of the haptic forces (F_{active}) intended to constrain specified degrees of freedom of movement of the end effector 22. As such, the control system 60 can also refine the active constraint control model (HMa) by adjusting parameters of the haptic object (Hobj) utilized by the active constraint control model (HMa). Such parameters can be geometrical parameters, mesh parameters, force parameters, and the like. For example, if during constraint of the end effector 22 to a virtual trajectory, the interaction style determines that the surgeon has tendency to aggressively reorient the end effector 22 away from the trajectory, the haptic object (Hobj) can be more tightly constrained to account for this tendency to keep the end effector 22 aligned to the virtual trajectory. If no active constraint control model (HMa) exists before determination of the user interaction style, the control system 60 can define (instead of to refine) the active constraint control model (HMa) and/or parameters of the haptic object (Hobj). Moreover, instead of refining/defining the active constraint control model (HMa), the control system 60 may be configured to define/refine the active constraint haptic forces (F_{active}), e.g., to adjust the stiffness or damping.

The control system 60 can perform various actions to refine the boundary constraint control model (HMb) based on the interaction style. For example, the control system 60 can adjust constraint parameters of any one or more virtual boundaries 71. The constraint parameters can be stiffness/damping parameters of any of the virtual boundaries 71. In another example, constraint parameters can be impulse parameters of any of the virtual boundaries 71. As described, the haptic object (Hobj) can be utilized to define the virtual boundaries 71 utilized by the boundary constraint control model (HMb). As such, the control system 60 can also refine the boundary constraint control model (HMb) by adjusting parameters of the haptic object (Hobj). Such parameters can be geometrical parameters, mesh parameters, force parameters, and the like. For example, if during the cutting of a plane for TKA, the interaction style determines that the surgeon has tendency to rotate the saw blade, the haptic object (Hobj) can be extended (widened) to account for this tendency. In some cases, the control system 60 can refine the boundary constraint control model (HMa) by re-specifying the degrees of freedom of movement that are to be constrained for the end effector 22. If no boundary constraint control model (HMb) exists before determination of the user interaction style, the control system 60 can define (instead of to refine) the boundary constraint control model (HMb) and/or parameters of the haptic object (Hobj). Moreover, instead of refining/defining the boundary constraint control model (HMb), the control system 60 may be configured to define/refine the boundary constraint haptic forces (F_{reactive}), e.g., to adjust the stiffness or damping.

The control system 60 can also perform various actions to refine the predefined arm feel control model (HMf) based on the interaction style. For example, the control system 60 can adjust a virtual mass of a virtual rigid body model of the end effector 22. When the virtual mass is increased, the end effector 22 will feel more sluggish. When the virtual mass is decreased, the end effector 22 will feel more responsive. The virtual mass can be processed by the virtual simulation run by the behavior controller 74 and is suitable for admittance control of the manipulator 14. In another example, the control system 60 can adjust damping parameters of the robotic arm in the predefined arm feel control model (HMf). For example, a higher damping will cause the manipulator 14 to feel more sluggish and a lower damping will cause the manipulator 14 to feel more responsive. Such modifications can be applied to admittance or impedance type control of the manipulator 14. If no boundary predefined arm feel control model (HMf)) exists before determination of the user interaction style, the control system 60 can define (instead of to refine) the damping parameters of the predefined arm feel control model (HMf) and/or the virtual mass. Moreover, instead of refining/defining the predefined arm feel control model (HMf), the control system 60 may be configured to define/refine the arm feel haptic forces (F_{damp}).

The machine learning model (MLM) may be configured to output behavior policy definitions/refinements as continuous action values, e.g., any haptic force refinement value within a continuous action space. In another implementation, machine learning model (MLM) outputs behavior policy definitions/refinements as a probability distribution, e.g., a probability of selecting each available action.

In some cases, the control system 60 is configured to store one or more updated behavior policies in non-transitory memory and retrieve the updated behavior policies for a subsequent collaborative interaction between the human and the manipulator 14. The control system 60 can retrieve such behavior policies based on user preferences or selections, or automatically and in response to detecting certain conditions or configuration attributes. For example, the machine learning model (MLM) can identify, over the course of many procedures, that a particular surgeon typically exhibits N different interaction styles which occur at various times or under different conditions. One interaction style can be related a particular surgical step (e.g., reaming). Another interaction style can be related to a particular robotic control mode (e.g., manual mode interaction with virtual boundaries), and so on. These interaction styles for the particular surgeon can be saved in memory (locally or in a remote server). Depending on the configuration attributes, any number of interaction styles can be determined for a particular individual. Then, when the surgeon uses the manipulator 14 for a subsequent procedure, the control system 60 can be configured to automatically update the behavior policy in response to detection of the surgical step or robotic control mode. This would enable immediate deployment of the behavior policy because the interaction styles and their contexts have already been determined. In another example, when the surgeon subsequently uses the manipulator 14, the control system 60 can display to the surgeon (e.g., using the display 38 and clinical application CA) a list of interaction styles (with contextual information) that have previously determined for the particular surgeon. The surgeon can then manually select/deselect the interaction styles they prefer/do not prefer. The behavior policies for the selected interaction styles can then be loaded during the procedure at the appropriate time.

Based on the determined interaction style, the control system 60 controls the manipulator 14 according to the defined/refined behavior policy. In turn, the control system 60 will provide the respective defined/refined haptic forces (F_{active}, F_{reactive}, F_{damp}) to the manipulator control 26 to compute joint torque (τ), which when combined with the feed forward torques (τ_{ff}), result in the total commanded torque (τₜ) by which the control system 60 will command the joints. Implementation of the interaction style may, or may not, be immediately experienced by the human user. For example, the human user may not experience the effects of interaction style implementation until the human user exhibits the behavior that the interaction style predicted. On the other hand, the human user may immediately feel the effects of interaction style implementation if the defined/refined behavior policy is such that it will affect any motion of the end effector 22.

The described machine learning models (MLM) can be configured to be limited in their use, e.g., for safety purposes or regulatory compliance. For instance, the described machine learning models (MLM) may be intentionally limited to specific motions or tasks or used only during the presence of certain conditions or during specified times. The control system 60 can maintain (standard) haptic control schemes if the machine learning model (MLM) is temporarily deactivated or turned off. The timing and conditions of activation of the described machine learning models (MLM) can be controlled or scheduled by a human (e.g., surgeon, staff, robotic designer) or can be automatically regulated by the control system 60.

As will be described in the following examples, the machine learning model (MLM) adapted for determining the interaction style can be described using various architectures, such as, but not limited to: model-free adaptive control (MFAC), model predictive control (MPC), and reinforcement learning (RL), and equivalents or combinations thereof. Any of these machine learning frameworks may be realized as a data-driven system. These machine-learning based frameworks aim at learning what is the best behavior policy for the given circumstances based on the behavior learned from the environment and understanding how the environment responds to certain haptic control policies. The control schemes of FIGS. 8 and 10 can be understood as a functional generalization that can embody any of the following examples. The proposed solutions are not limited to any particular example described herein and may be captured more as generally set forth.

### c. Model-Free Adaptive Control

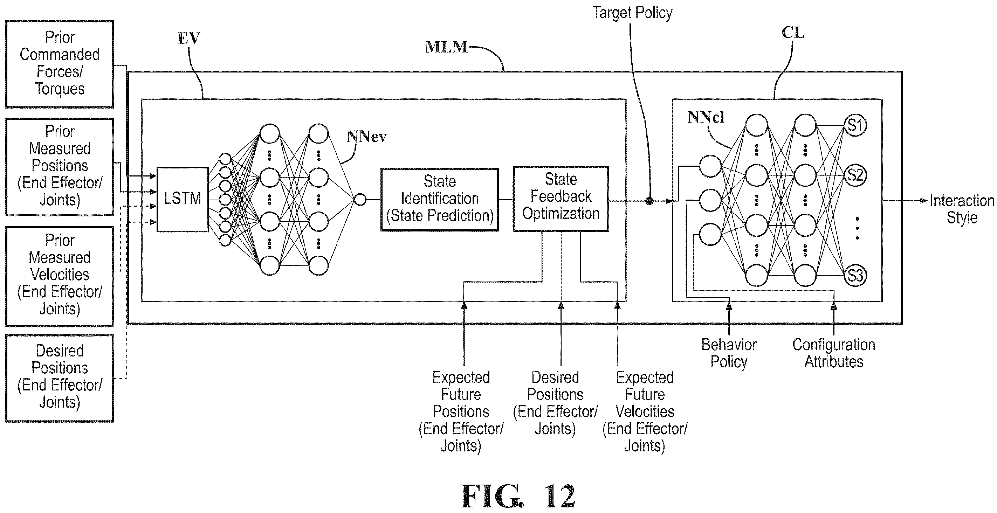

In one implementation, and with reference to FIG. 12, the machine learning model (MLM) is realized using a form of model-free adaptive control (MFAC) to determine the interaction style. Model-free adaptive control enables the control system 60 to adjust the behavior policy dynamically, and in real-time, without requiring a detailed mathematical model of the state of the manipulator 14 or the environment in which the manipulator 14 interacts. As such, MFAC can be implemented as a data-driven control method. MFAC can adaptively and autonomously learn, without requiring complicated trial and error tuning procedures. MFAC is also mathematically proven to provide stability to a closed loop system. MFAC also can operate using limited robotic data. Using MFAC, the machine learning model (MLM) can reconstruct the states of the manipulator 14 from the measured input data and the control policy can be designed based on the linear or non-linear state feedback control theory.

Referring to FIG. 12, one example of MFAC is illustrated wherein the evaluator (EV) is realized as a model identification and optimization block. In this example, the evaluator (EV) is implemented with the evaluator neural network (NNev), such as a deep-learning network. The neural network (NNev) can receive, at the input layer, the robotic data, including the prior commanded forces/torques and prior measured positions/poses of the end effector 22 and/or joints (J). The evaluator (EV) can optionally also receive the prior measured velocities (of the end effector and/or joints) as well as the desired poses/positions (of the end effector and/or joints). The robotic data can optionally be pre-processed by the LSTM before being injected into the neural network (NNev). The neural network (NNev) processes the values to generate an output that comprises an identification of one or more state(s) of the manipulator 14 (as shown at the "state identification" block).

When the full dynamics of a linear robotic system are known, state feedback can be applied to achieve the optimal performance for the robotic system. However, it may not be feasible to know the full dynamics of the robotic system and the knowledge may be only partially available. In other scenarios, the robotic system knowledge may be limited to historic data of the output for the given known inputs. With the proposed MFAC architecture, the evaluator (EV), using the neural network (NNev), can identify a model of the robotic system based on the prior robotic data, and reconstruct the states of the system that can be used are used to find the target policy (state feedback control) by solving the optimization problem (set forth in equation 7 of FIG. 7). Notably, the identified state of the manipulator 14 may be identified in view of the human collaborative interaction, which can exhibit dynamic or non-linear behavior. The state of the manipulator 14 in this sense can be a state that is not otherwise easily identified or predicted based on the prior values and the neural network (NNev) can be used as a surrogate to a predetermined system model of the manipulator 14 to provide better identification of the new states. Although prior values are utilized, the identified state of the manipulator 14 are generated to account for changing conditions arising from uncertainties or non-linearities in the task space. In one example, the state of the manipulator identifies an actual or next tool pose, or an actual or next constraint pose for the tool. This state identification can be then utilized to construct a more efficient target policy when the manipulator 14 dynamics are partially known (grey box) or fully unknown (black box).

The evaluator (EV) can automatically identify, generate, or update the target policy. Namely, the identified states can be subjected to optimization based on expected/future poses/positions (end effector/joints), the desired poses/positions (end effector/joints), and expected/future velocities (end effector/joints). Optimization can also include the cost function (CF). In some cases, the result of this optimization can also be used to derive a tuning parameter to adjust the weights and biases of the neural network (NNev), thereby providing real-time feedback enabling the neural network (NNev) to optimize its identification policy to adjust to changing conditions.

The target policy is then utilized by the classifier (CL). The classifier (CL) implements the neural network (NNcl) which can take as an input the target policy, as well as the behavior policy, and the one or more configuration attributes. The classifier neural network (NNcl) determines the interaction style of the user, as described above. Depending on the nature of the interaction style, the behavior policy or the haptic forces can be defined/refined using any of the techniques described above.

### d. Model-Predictive Control

In another implementation, also represented by FIG. 12, the machine learning model (MLM) is realized using a form of model predictive control (MPC) to determine the interaction style. MPC is configured to forecast control decisions by utilizing real-time (or prior) input-output data to make predictions and determine the target policy. Like MFAC, the MPC can be implemented using data-driven control, and therefore, can operate without requiring a detailed mathematical model of the state of the manipulator 14 or the environment in which the manipulator 14 interacts. Using MPC, the control system 60 can adaptively and autonomously learn environmental interactions over time. MPC similarly can operate using the robotic data and desired values described above for MFAC. However, instead of the evaluator (EV) identifying the model of the robotic system, the evaluator (EV) predicts the model of the system (state prediction). The predicted state feedback optimization and target policy generation can be like that described for MFAC. The classifier (CF) processes the target policy and other inputs to determine the interaction style, as described.

### e. Reinforcement Learning

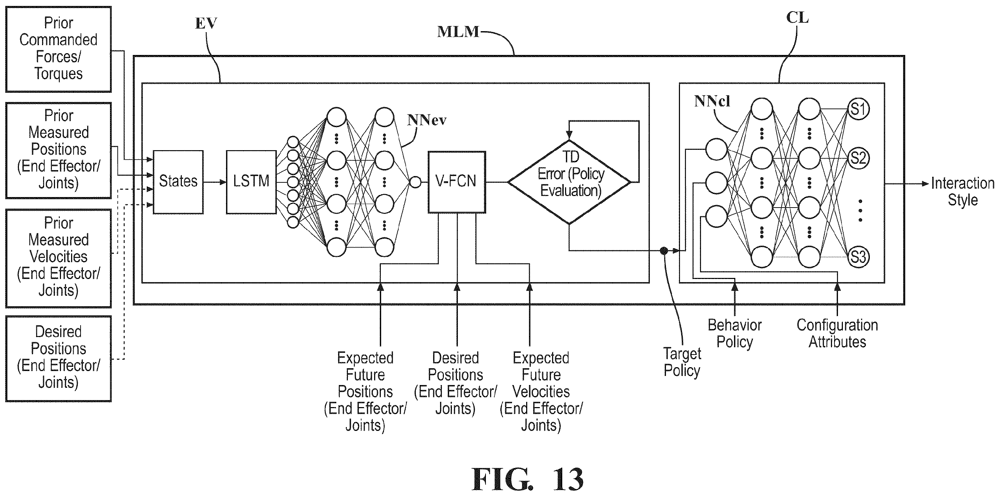

In another implementation, and with reference to FIG. 13, the machine learning model (MLM) is realized using a form of reinforcement learning (RL) to determine the interaction style. Reinforcement learning is well-suited to compute the near optimal solutions for partially to fully unknown dynamic robotic systems. Reinforcement learning can be utilized to evaluate the current behavior policy and obtain the target policy by evaluating the prior robotic data.

The RL model may be on-policy or off-policy. On-policy reinforcement learning treats the target policy and the behavior policy as the same. On the other hand, off-policy reinforcement learning treats the target policy and the behavior policy as independent. The RL model described herein can be implemented in various forms, including but not limited to: an Integral Reinforcement Learning (IRL) model, Deep Deterministic Policy Gradient (DDPG) model, Actor-Critic model, or Advantage Actor-Critic (A2C) models. IRL and DDPG models are both off-policy RL techniques that use experience replay to learn optimal control solutions. IRL computes a "value function" that estimates the expected long-term reward an agent can achieve from a given state. On the other hand, DDPG is a Q-learning technique that is a specific algorithm used to learn the "Q-value function," which estimates the expected long-term reward for taking a particular action in a specific state. Both IRL and DDPG algorithms can be implemented with the actor-critic networks where the actor applies a continuous control while the critic network incrementally corrects the actor's behavior until the optimal performance is achieved.

The specific architecture in FIG. 13 can be seen as an actor-critic network where the critic network evaluates the optimal IRL target policy for the unknown dynamics. In FIG. 13, one example of a RL model is illustrated wherein the evaluator (EV) is realized as the critic network and the classifier (CL) is realized as the actor network. The evaluator (EV) critic network seeks to generate the target policy and evaluate the quality of the target policy, thereby providing feedback to the classifier (CL) actor network. A primary difference between the architecture of FIG. 13 compared to the classical actor-critic network is that the actor network utilizes the IRL target policy and a machine learning approach to determine the interaction style during robotics-assisted surgical/medical application. Then, another policy update can be applied to the arm control based on the classified style which is different than the common actor-critic networks where the critic network directly updates the actor policy.

In the illustrated example, the evaluator (EV) is implemented with a neural network (NNev), such as a deep-learning neural network. The neural network (NNev) can receive, at the input layer, the robotic data, including the prior commanded forces/torques and prior measured positions/poses of the end effector 22 and/or joints (J). The evaluator (EV) can optionally also receive the prior measured velocities (of the end effector and/or joints) as well as the desired poses/positions (of the end effector and/or joints). The robotic data can optionally be pre-processed by the LSTM before being injected into the neural network (NNev). These values may be individually inputted to the neural network (NNev) or may be combined prior to input to the to the neural network (NNev). More specifically, these values may be inputted into input node(s) that are configured to receive the states of the manipulator 14 and/or end effector 22. The state in this example can be like any of the examples of the states of the manipulator 14 and/or end effector 22 described above. In other examples, the evaluator (EV) may be implemented to perform IRL without a neural network, e.g., by integrating the reward signal over time.

The neural network (NNev) processes the values to generate an output, which when combined with expected/future poses/positions (end effector/joints), the desired poses/positions (end effector/joints), and expected/future velocities (end effector/joints), is used to compute V-FCN or the "value function". The V-FCN estimates the expected long-term reward an agent can achieve from a given state. The target policy in this example can be achieved by utilizing the value function technique (to maximize the expected long-term reward the robotic system can achieve from a given state). The neural network (NNev) can make the value function estimation by considering the reward received from the interaction. Optionally, the cost function (CF) can be utilized as a reward function. The cost function (CF) can be utilized to measure the model error and guide the learning of the neural network (NNev) to adapt to changing conditions. The evaluator (EV) critic network may perceive a reward from the environment if the previous action contributed to resolving steady state error. The evaluator (EV) critic network may seek to maximize the return (its cumulative reward) and obtain an optimal target policy. Optionally, the result of this function estimation can also be used to derive a tuning parameter to adjust the weights and biases of the neural network (NNev), thereby providing real-time feedback enabling the neural network (NNev) to optimize the value function to adjust to changing conditions.

The classifier (CL) actor network receives the target policy from the evaluator (EV) critic network. The classifier (CL) actor network also can receive the behavior policy and configuration attributes as an input, as described above. The classifier (CL) actor network implements neural network (NNcl) that processes these inputs to determine the interaction style. In one optional implementation, because the classifier (CL) actor network determines the interaction style based on the states and target control policy, the classifier (CL) actor network can establish its own policy to map the states to actions. In some cases, the classifier (CL) actor network can derive, from this policy, a tuning parameter to adjust the weights and biases of its neural network (NNcl), thereby providing real-time feedback enabling the neural network (NNev) to optimize its policy to adjust to changing conditions and enable adaptive prediction to generate the best action.

Several embodiments have been described in the foregoing description. However, the embodiments discussed herein are not intended to be exhaustive or limit the invention to any particular form. The terminology, which has been utilized, is intended to be in the nature of words of description rather than of limitation. Many modifications and variations are possible in light of the above teachings and the invention may be practiced otherwise than as specifically described.

## Claims

1. A surgical or medical system (10) comprising:
a manipulator (14) comprising a robotic arm including a plurality of links (18) and joints (J) and an end effector (22) supported and moveable by the robotic arm, wherein the end effector (22) is adapted to facilitate collaborative interaction between a human and the manipulator (14); and
a control system (60) coupled to the manipulator (14) and being configured to:
control the manipulator (14) according to a behavior policy that defines a physical feel of the manipulator (14) for the human;
acquire robotic data from the manipulator (14) in response to collaborative interaction between the human and the manipulator (14);
implement a machine learning model (MLM) that is configured to receive and process the robotic data to determine an interaction style of the human in collaborating with the manipulator (14);
update the behavior policy based on the determined interaction style to customize the physical feel of the manipulator (14) to the determined interaction style; and
control the manipulator (14) according to the updated behavior policy.

2. The surgical or medical system (10) of claim 1, wherein:
the control system (60) implements one or more haptic control models (HMa, HMb, HMf) that are configured to compute haptic forces that are intended to constrain movement of the end effector (22) in a task space of the manipulator (14);
the behavior policy is defined based on the one or more haptic control models (HMa, HMb, HMf); and
the control system (60) updates the behavior policy based on the determined interaction style by being configured to update the one or more haptic control models (HMa, HMb, HMf).

3. The surgical or medical system (10) of claim 2, wherein:
the one or more haptic control models (HMa, HMb, HMf) include an active constraint control model (HMa) configured to compute haptic forces that are intended to constrain specified degrees of freedom of movement of the end effector (22);
the behavior policy is defined, at least in part, based on the active constraint control model (HMa); and
the control system (60) updates the behavior policy based on the determined interaction style by being configured to update the active constraint control model (HMa);
and optionally;
wherein the control system (60) updates the active constraint control model (HMa) by being configured to perform one or more of:
adjust constraint parameters of any of the specified degrees of freedom;
adjust geometrical parameters of a haptic object (Hobj) utilized by the active constraint control model (HMa) in computation of the haptic forces that are intended to constrain specified degrees of freedom of movement of the end effector (22); and/or
re-specify the degrees of freedom of movement that are to be constrained for the end effector (22).

4. The surgical or medical system (10) of any one of claims 2 to 3, wherein:
the one or more haptic control models (HMa, HMb, HMf) include a boundary constraint control model (HMb) configured to compute haptic forces that are intended to constrain movement of the end effector (22) relative to a virtual boundary (71);
the behavior policy is defined, at least in part, based on the boundary constraint control model (HMb); and
the control system (60) updates the behavior policy based on the determined interaction style by being configured to update the boundary constraint control model (HMb);
and optionally;
wherein the control system (60) updates the boundary constraint control model (HMb) by being configured to perform one or more of:
adjust constraint parameters of the virtual boundary (71); and/or
adjust geometrical parameters of a haptic object (Hobj) utilized by the boundary constraint control model (HMb) in computation of the haptic forces that are intended to constrain movement of the end effector (22) relative to the virtual boundary (71).

5. The surgical or medical system (10) of any one of claims 2 to 4, wherein:
the one or more haptic control models (HMa, HMb, HMf) include a predefined arm feel control model (HMf) configured to compute haptic forces that are intended to define a baseline physical feel of the manipulator (14) to the human;
the behavior policy is defined, at least in part, based on the predefined arm feel control model (HMf); and
the control system (60) updates the behavior policy based on the determined interaction style by being configured to update the predefined arm feel control model (HMf);
and optionally;
wherein the control system (60) updates the predefined arm feel control model (HMt) by being configured to:
adjust a virtual mass of a virtual rigid body model of the end effector (22); and/or adjust damping parameters of the robotic arm.

6. The surgical or medical system (10) of any preceding claim, wherein:
the robotic data comprises one or more of:
prior commanded forces applied to the end effector (22);
prior commanded torques applied to the joints (J) of the robotic arm;
prior measured poses of the end effector (22); and
prior measured positions of the joints (J) of the robotic arm;
and optionally;
the robotic data further comprises one or both of:
prior measured velocities of the end effector (22); and
prior measured velocities of the joints (J) of the robotic arm.

7. The surgical or medical system (10) of any preceding claim, wherein the machine learning model (MLM) is configured to:
determine a target policy based on the robotic data; and
compare the behavior policy and the target policy to determine the interaction style of the human in collaborating with the manipulator (14).

8. The surgical or medical system (10) of claim 7, wherein:
the control system (60) is configured to: predict future robotic data; obtain desired positions of the end effector (22) and/or the joints (J) of the robotic arm; and input the future robotic data and the desired positions into the machine learning model (MLM); and
the machine learning model (MLM) is configured to determine the target policy by optimizing a cost function (CF) based on the future robotic data and the desired positions.

9. The surgical or medical system (10) of claim 7, wherein the machine learning model (MLM) comprises:
a first neural network (NNev) configured to receive and process the robotic data and determine the target policy based on the robotic data;
and optionally;
a second neural network (NNcl) configured to:
receive, as first input, the target policy determined by the first neural network (NNev);
receive, as a second input, one or more configuration attributes (CA) defining a context of the collaborative interaction between the human and the manipulator (14);
receive, as a third input, the behavior policy;
compare the behavior policy and the target policy in view of the context defined by the one or more configuration attributes (CA); and
based on the comparison, determine the interaction style of the human in collaborating with the manipulator (14).

10. The surgical or medical system (10) of any preceding claim, wherein the human is a surgeon, and:
the end effector (22) is a surgical tool that is configured to surgically manipulate an anatomy (A) of a patient (12);
the surgical tool is adapted to facilitate collaborative interaction between an arm of the surgeon and the manipulator (14); and
the control system (60) is configured to implement the machine learning model (MLM) to determine the interaction style of the surgeon in collaborating with the manipulator (14).

11. The surgical or medical system (10) of any one of claims 1-9, wherein the human is a patient, and:
the end effector (22) is a limb holder that is configured to couple to a limb of the patient;
the limb holder is adapted to facilitate collaborative interaction between the limb of the patient and the manipulator (14); and
the control system (60) is configured to implement the machine learning model (MLM) to determine the interaction style of the patient in collaborating with the manipulator (14).

12. The surgical or medical system (10) of any preceding claim, wherein the control system (60) is configured to store the updated behavior policy in non-transitory memory and retrieve the updated behavior policy for a subsequent collaborative interaction between the human and the manipulator (14).

13. The surgical or medical system (10) of any preceding claim, wherein the control system (60) is configured to:
implement the machine learning model (MLM) to determine a first interaction style of the human in context of a first condition;
implement the machine learning model (MLM) to determine a second interaction style of the human in context of a second condition;
determine a first updated behavior policy based on the first interaction style;
determine a second updated behavior policy based on the second interaction style;
store the first updated behavior policy and the second updated behavior policy in non-transitory memory; and
for a subsequent collaborative interaction between the human and the manipulator (14);
automatically retrieve from the non-transitory memory and implement the first updated behavior policy in response to detection of the first condition; and
automatically retrieve from the non-transitory memory and implement the second updated behavior policy in response to detection of the second condition.

14. A computer-implemented method of operating the surgical or medical system (10) of any preceding claim.

15. A computer program product configured for use with a surgical or medical system (10), the surgical or medical system (10) including a manipulator (14) including a robotic arm with a plurality of links (18) and joints (J) and an end effector (22) supported and moveable by the robotic arm, the end effector (22) being adapted to facilitate collaborative interaction between a human and the manipulator (14), and one or more processors coupled with the manipulator (14), the computer program product comprising instructions, which when executed by the one or more processors, are configured to:
control the manipulator (14) according to a behavior policy that defines a physical feel of the manipulator (14) for the human;
acquire robotic data from the manipulator (14) in response to collaborative interaction between the human and the manipulator (14);
implement a machine learning model (MLM) that is configured to receive and process the robotic data to determine an interaction style of the human in collaborating with the manipulator (14);
update the behavior policy based on the determined interaction style to customize the physical feel of the manipulator (14) to the determined interaction style; and
control the manipulator (14) according to the updated behavior policy.
